# EUROPEAN PATENT APPLICATION

(11) **EP 4 721 753 A1**
(43) Date of publication of application: **08.04.2026**
(21) Application number: 24815857.8
(22) Date of filing: 29.05.2024
(51) Int. Cl.: A61K 38/16, A61K 31/7088, A61P 31/14, A61K 38/10

(54) **PHARMACEUTICAL COMPOSITION FOR PREVENTING OR TREATING CORONAVIRUS INFECTIOUS DISEASES COMPRISING CELL-PERMEABLE PEPTIDE-NUCLEIC ACID OLIGOMER AS ACTIVE INGREDIENT**

(30) Priority: 01.06.2023 KR 20230070987
(71) Applicant: GiftedMS Co., Ltd., Incheon 21983 (KR)
(72) Inventor: KIM, Sung Chun, Seoul 04426 (KR); JUNG, Daram, Seongnam-si Gyeonggi-do 13597 (KR); JEON, Yea Sel, Yongin-si Gyeonggi-do 17079 (KR)
(74) Representative: Russell, Tim
(86) International application number: PCT/KR2024/007331
(87) International publication number: WO 2024/248482

(57) **Abstract**

The present invention provides a use of a PNA oligomer comprising a modified PNA for the prevention or treatment of coronavirus infectious diseases. The PNA oligomer of the present invention can be delivered into virus-infected cells without a delivery vehicle and inhibit viral proliferation, and thus has no side effects caused by a delivery vehicle for increasing the cell permeability of conventional drugs, and can inhibit viral proliferation by binding to a target even when there is a mismatch, due to its high binding affinity to the target. Therefore, it is expected that the PNA oligomer will be used as an effective coronavirus therapeutic agent that can respond to rapidly mutating virus infectious diseases.

## Description

### [Technical Field]

The present invention relates to a pharmaceutical composition for preventing or treating coronavirus infectious diseases, including, as an active ingredient, a peptide-nucleic acid oligomer that targets the genomic positive (+) and/or negative (-) RNA of coronavirus.

### [Background Art]

Coronaviruses usually cause enzootic infections in animals such as birds and mammals, but they can also cross the animal-human species barrier, leading to zoonotic infections of humans. Coronaviruses cause various diseases including respiratory, gastrointestinal, and central nervous system infections in mammals, birds, and humans, and various coronaviruses causing human and animal diseases have emerged over the past 50 years. Coronaviruses also cause various diseases in livestock, particularly those affecting agriculture, and after acute respiratory infections were first reported in chickens raised as livestock in North America in the late 1920s, Edward C. Kendall, Malcolm Bynoe, and David Tyrrell identified coronaviruses as one of the causes of the common cold in animals in 1961, and named it coronavirus B814. After many studies, it was first discovered in the UK in 1965 that coronaviruses are one of the causes of respiratory infections in humans, and virologist June Almeida and physician David Tyrrell named it coronavirus in 1967, and published the discovery under the name coronavirus in the journal Nature in 1968. Since the 1970s, it has been studied using betacoronavirus, murine hepatitis virus (MHV), and human alphacoronavirus HCoV-229E models for about 20 years. Human circulating coronaviruses (HCoVs) HCoV-229E, HCoV-NL63, HCoV-OC43, and HCoV-HKU1 generally cause mild upper respiratory diseases, and are collectively associated with 10 to 30% of common cold patients. Coronaviruses, which were previously known only as one of the causes of the common cold, have formed several mutations of Middle East respiratory syndrome (MERS) and now SARS-CoV-2, which causes severe acute respiratory syndrome type 2 (COVID-19), since the outbreak of severe acute respiratory syndrome (SARS) in 2002, causing new infectious diseases such as severe infectious pneumonia in humans.

Coronaviruses (CoVs) are spherical, enveloped RNA viruses with a diameter of about 80 to 125 nm, whose envelope is derived from the plasma membrane of the host cell. The viral genome is present in and protected by a helical capsid formed by the nucleocapsid protein (N), which is also surrounded by the envelope. The envelope has three protruding viral structural proteins: spike protein (S), membrane protein (M), and envelope protein (E), and includes the nucleocapsid protein inside the membrane.

The coronavirus genome is a non-segmented, single-stranded, positive-sense RNA measuring 26 to 32 kilobases in size, making it the largest genome of all RNA viruses.

Meanwhile, antiviral drugs treat viral infectious diseases by weakening or eliminating the action of viruses that have invaded the human body. Antiviral drugs are classified into methods that directly recognize and attack viral proteins, methods that inhibit each stage of the viral life cycle to prevent the viral replication process, methods that enhance immunity, and the like.

Antisense oligonucleotides (ASOs) that specifically bind to a viral genome can inhibit the synthesis of viral proteins and inhibit their proliferation, but ASOs composed of DNA and/or RNA have low stability and are easily degraded by nucleases present in the body, limiting their therapeutic use and there is also a problem in that a delivery vehicle is required for their introduction into cells.

The present inventors have conducted extensive research to develop a therapeutic agent for coronavirus infectious diseases using a peptide-nucleic acid derivative that can move into cells without a delivery vehicle and is highly stable, thereby completing the present invention.

### [Disclosure]

### [Technical Problem]

A technical problem achieved by the present invention is to provide a PNA oligomer including a modified PNA derivative for use in the prevention and/or treatment of coronavirus infectious diseases.

However, the technical problems to be achieved by the present invention are not limited to the aforementioned problems, and other problems that are not mentioned may be clearly understood by those skilled in the art from the following description.

### [Technical Solution]

To solve the above problem, the present invention provides a pharmaceutical composition for preventing or treating coronavirus infectious diseases, including a peptide nucleic acid (PNA) oligomer represented by the following Chemical Formula 1 or a pharmaceutically acceptable salt thereof as an active ingredient.

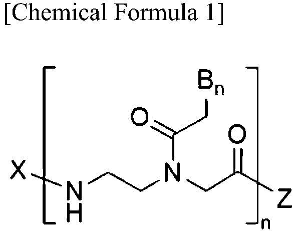

In the present invention, the PNA oligomer can complementarily bind to a portion of the genomic (+) RNA or genomic (-) RNA of coronavirus, and has a length of 10 to 30 mers.
Specifically, in Chemical Formula 1, n is an integer between 10 and 30;
X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and a substituted or non-substituted arylsulfonyl;
Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl;
B1, B2, ......, Bn-1 and Bn are each independently selected from a natural nucleotide and a non-natural nucleotide of adenine (A), guanine (G), thymine (T), or cytosine (C);
one or more of B1 to Bn are modified PNAs, and specifically, one or more of B1 to Bn are each independently selected from the group consisting of modified PNAs of the following Chemical Formulae 2 to 4; 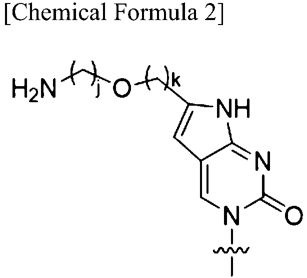 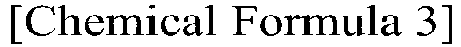 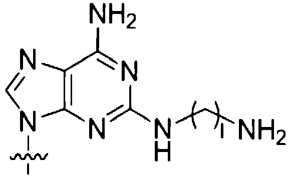 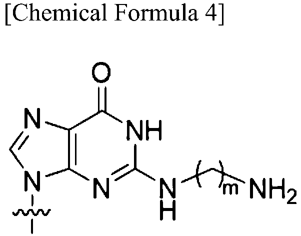
in Chemical Formulae 2 to 4, j, k, l, and m are each independently an integer between 1 and 16.

Meanwhile, the pharmaceutical composition of the present invention does not include a delivery vehicle for intracellular delivery of the PNA oligomer.

In one embodiment of the present invention, in Chemical Formula 1, X may be Fmoc [(9-fluorenyl)methyloxy]carbonyl], Fethoc [2-(9-fluorenyl)ethyl-1-oxy]carbonyl], acetyl (Ac), benzoyl, or hydrogen, and more specifically, may be Fethoc-.

In another embodiment of the present invention, in Chemical Formula 1, X may be Fethoc, Z may be NH2, j may be 2, k may be 1, l may be 5, and m may be 6.

In still another embodiment of the present invention, the coronavirus may be one or more selected from the group consisting of human coronavirus 229E (HCoV-229E), human coronavirus OC43 (HCoV-OC43), severe acute respiratory syndrome coronavirus (SARS-CoV), human coronavirus NL63 (HCoV-NL63), human coronavirus HKU1 (HCoV-HKU1), Middle East respiratory syndrome coronavirus (MERS-CoV), and severe acute respiratory syndrome coronavirus type 2 (SARS-Cov-2).

Since the present invention has constructed a PNA oligomer complementary to HCoV-OC43 RNA in the following Table 1 and confirmed its viral proliferation inhibitory effect, in yet another embodiment of the present invention, the PNA oligomer may be one or more selected from the group consisting of PNA oligomers in Table 1, and the coronavirus may be HCoV-OC43, but the target virus is not limited thereto.

Since the present invention has constructed a PNA oligomer complementary to SARS-CoV-2 in the following Table 2 and confirmed its viral proliferation inhibitory effect, in yet another embodiment of the present invention, the PNA oligomer may be one or more selected from the group consisting of PNA oligomers in Table 2, and the coronavirus may be SARS-CoV-2, but the target virus is not limited thereto.

Meanwhile, since the present invention randomly selected a PNA oligomer from the PNA oligomers in Table 1 to confirm whether it can be applied to inhibiting the proliferation of SARS-CoV-2 virus, and as a result, it was confirmed that the selected PNA oligomer forms a complementary binding region including partial mismatches with the SARS-CoV-2 genome and inhibits the expression of SARS-CoV-2 proteins, in yet another embodiment of the present invention, the PNA oligomer may be 80% or more complementary to a portion of the genomic (+) RNA or genomic (-) RNA of coronavirus.

Further, the present invention provides a method for preventing or treating coronavirus infectious diseases and a method for inhibiting coronavirus proliferation, the methods including administering the above-described PNA oligomer to an individual.

In one embodiment of the present invention, the individual may be a mammal infected with coronavirus or at risk of exposure to the coronavirus.

In another embodiment of the present invention, each of the methods may further include detecting coronavirus in a biological sample isolated from the individual after administration of the PNA oligomer.

In addition, the present invention provides a method for inhibiting coronavirus proliferation, the method including treating cells with the above-described PNA oligomer.

In one embodiment of the present invention, the cells may be mammalian cells.

In another embodiment of the present invention, the cells may be coronavirus-infected cells.

In still another embodiment of the present invention, the method may further include lysing cells after PNA oligomer treatment and detecting a coronavirus genetic material.

In yet another embodiment of the present invention, the method may be used to separately prepare cells that have not been treated with a PNA oligomer as a control group to confirm the effect of PNA oligomer treatment on inhibiting coronavirus proliferation, and the cells in the control may be a cell line identical to the cells treated with the PNA oligomer.

In yet another embodiment of the present invention, the method may further include lysing cells treated with the PNA oligomer and cells of the same cell line not treated with the PNA oligomer, respectively, and quantifying the level of a coronavirus genetic material.

Furthermore, the present provides a use of the above-described PNA oligomer for the preparation of a drug for preventing or treating coronavirus.

### [Advantageous Effects]

The present invention provides a use of a PNA oligomer including a modified PNA for the prevention or treatment of coronavirus infectious diseases. The PNA oligomer of the present invention can be delivered into virus-infected cells without a delivery vehicle and inhibit viral proliferation, and thus has no side effects caused by a delivery vehicle for increasing the cell permeability of conventional drugs, and can inhibit viral proliferation by binding to a target even when there is a mismatch, due to its high binding affinity to the target. Therefore, it is expected that the PNA oligomer will be used as an effective coronavirus therapeutic agent that can respond to rapidly mutating virus infectious diseases.

### [Description of Drawings]

FIGS. 1A to 1C show some of the chromatogram results confirming that the designed PNA oligomers were constructed as intended. Specifically, FIG. 1A shows the C₁₈-reverse phase UPLC reverse phase chromatography results of PNA123 before HPLC purification, FIG. 1B shows the C₁₈-reverse phase UPLC reverse phase chromatography results of PNA123 after HPLC purification, and FIG. 1C shows the ESI-TOF mass spectrum results of PNA123 after C₁₈-RP prep HPLC.
FIG. 2 shows the results of the titer analysis of Paxlovid in the SARS-CoV-2 virus.
FIGS. 3A to 3I and 4A to 4K show the results of the titer analysis of PNA oligomers and their combination drugs in the SARS-CoV-2 virus.
FIG. 5 shows the results of western blot analysis confirming the effect of inhibiting the proliferation of SARS-CoV-2 virus using PNA oligomers complementary to a portion of HCoV-OC43 RNA.

### [Best Mode]

Coronavirus is a single-stranded RNA virus that synthesizes genomic positive (+) RNA into genomic negative (-) RNA using RNA polymerase to construct double-stranded RNA. The present inventors intended to block the replication process of the coronavirus using oligonucleotides that target the coronavirus genome.

However, since oligonucleotides composed of DNA and/or RNA have low stability and are easily degraded by nucleases present in an organism, there are limitations in using them for the use of treating viral infectious diseases. To overcome this, various types of non-natural oligonucleotides have been developed, and the present inventors have developed a new peptide nucleic acid (PNA) in a previous study.

PNA is a polypeptide with N-(2-aminoethyl)glycine [Aeg, N-(2-aminoethyl)glycine] as a unit backbone, and was invented by Nielsen et al. PNA can bind to complementary nucleic acids just like DNA and RNA, and the binding affinity of RNA-PNA is stronger than that of RNA-DNA or RNA-RNA. In addition, since PNA is structurally very different from DNA, it is not recognized by hepatic transporters that recognize DNA.

A PNA monomer has the 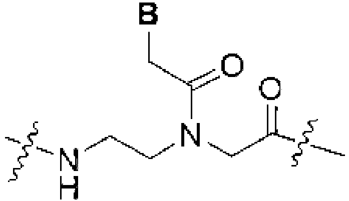 structure, and in the PNA monomer structure, B is a nucleobase, the nucleobase may be a natural or non-natural nucleobase, and in the present specification, examples of the natural or non-natural nucleobase are as follows.

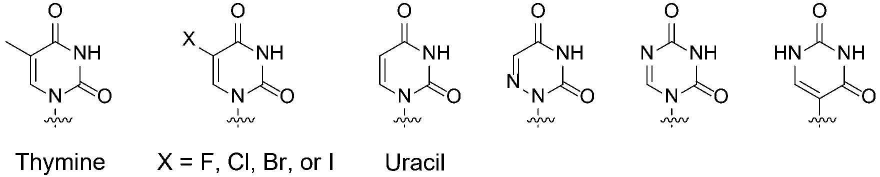

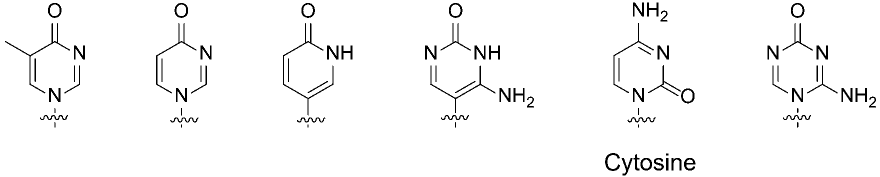

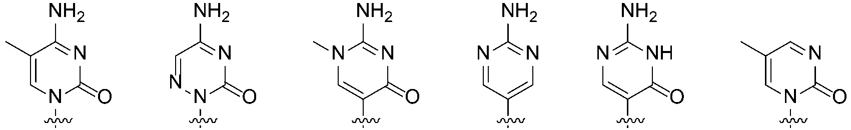

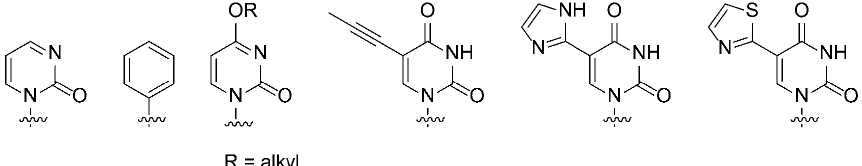

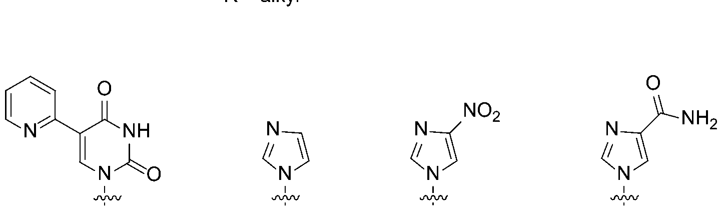

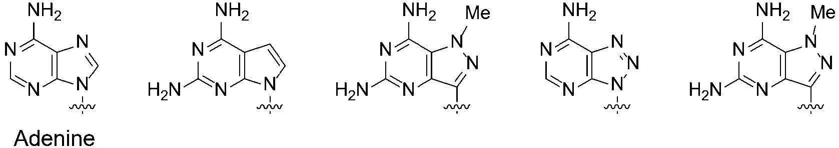

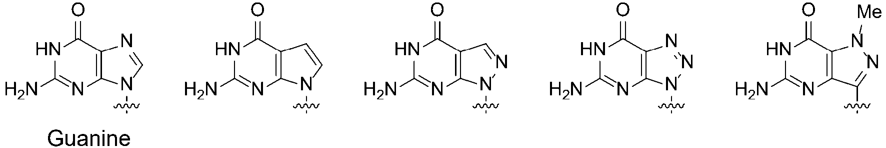

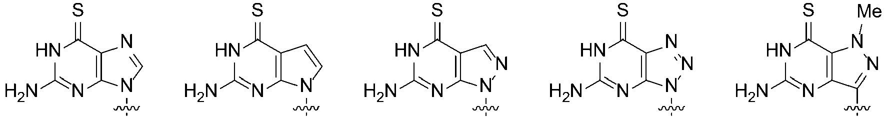

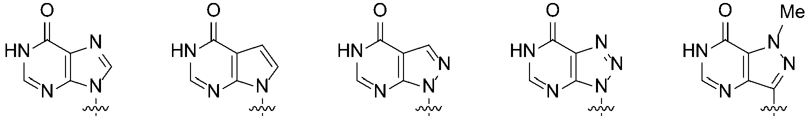

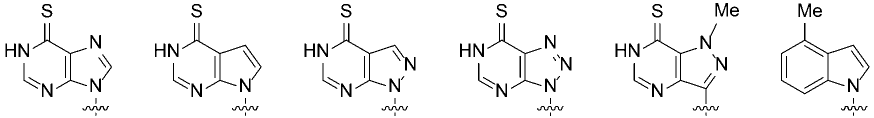

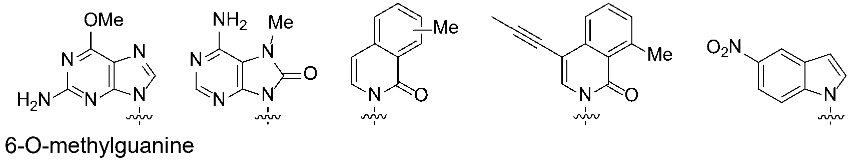

In a previous study, the present inventors constructed PNA using a "modified nucleobase" in which cationic lipids were covalently linked, and confirmed that the PNA including the modified nucleobase had high cell membrane permeability, thereby developing a PNA with increased cell permeability (PCT/KR2009/001256). However, there has been no study at all on whether the modified PNA can be used for the treatment of viral infectious diseases.

The modified nucleobases used in the present invention to construct the PNA with increased cell permeability are as follows.

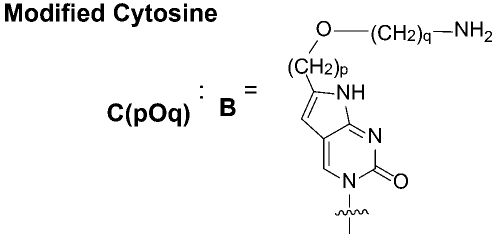

### Modified Adenine

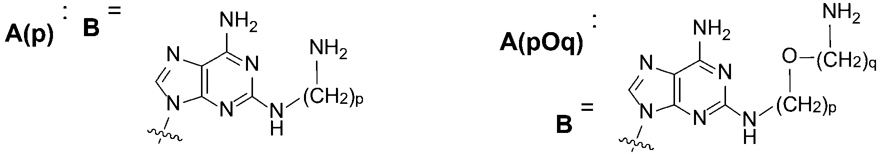

### Modified Guanine

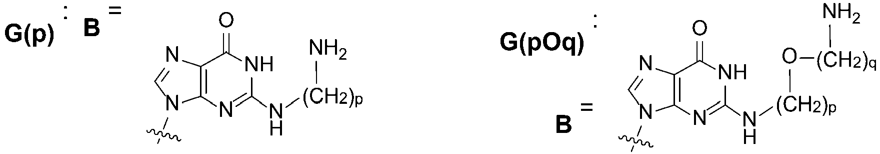

The modified nucleobase belongs to non-natural nucleobases, and in the present specification, the term "modified nucleobase" may be used interchangeably with the term "non-natural nucleobase" unless it is intended to distinguish it from non-natural nucleobases other than the modified nucleobase. In addition, in the present specification, when it is necessary to distinguish between a PNA including a modified nucleotide and a PNA not including the modified nucleotide, the PNA including the modified nucleotide is referred to as a "modified PNA."

The present inventors constructed a PNA oligomer of the following Chemical Formula 1 in which the above-described PNA monomers are covalently linked.

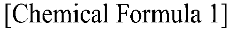

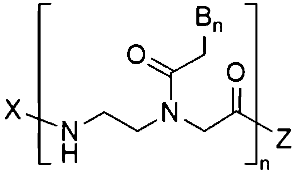

The PNA oligomer of Chemical Formula 1 was designed to be able to complementarily bind to a portion of the coronavirus genomic RNA of the coronavirus, and the complementary binding region was set to 10 mer or more.

Accordingly, in Chemical Formula 1, n is an integer between 10 and 30. This literally means that n is an integer selected from 10, 11,12, 13, 14, 15, 16, 17, 18, 19, 20, 21,22, 23, 24, 25, 26, 27, 28, 29, and 30.

In the present specification, the "complementary binding region" means a continuous region including the start and end of the bases where the coronavirus genomic RNA and the PNA oligomer form a base pair. Meanwhile, since the modified PNA has high binding affinity with the target gene, even when there is a mismatch in the complementary binding region, the PNA may bind to the target and inhibit viral replication. Therefore, in the present specification, "complementary binding" includes complete complementary binding and partial complementary binding with a mismatch of 20% or less within the range where the PNA derivative can maintain binding affinity to the target RNA. For example, when n=10 in Chemical Formula 1, the PNA oligomer of Chemical Formula 1 may include 1 or 2 mismatches in the complementary binding region with a portion of the coronavirus genomic RNA, when n=20, the PNA oligomer of Chemical Formula 1 may include 1 or 4 mismatches in the complementary binding region with a portion of the coronavirus genomic RNA, and when n=30, the PNA oligomer of Chemical Formula 1 may include 1 or 6 mismatches in the complementary binding region with a portion of the coronavirus genomic RNA. In this case, the mismatches may be continuous and/or discontinuous.

In Chemical Formula 1, X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and substituted or non-substituted arylsulfonyl.

In Chemical Formula 1, Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl.

Substituents for the description of the PNA derivative of Chemical Formula 1 are described below.

Examples of the substituted or non-substituted alkyl group are as follows.

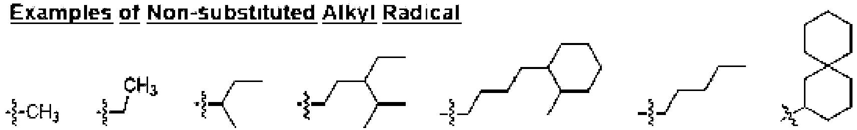

### Examples of Substituted Alkyl Radical

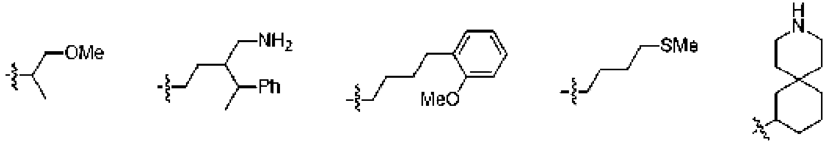

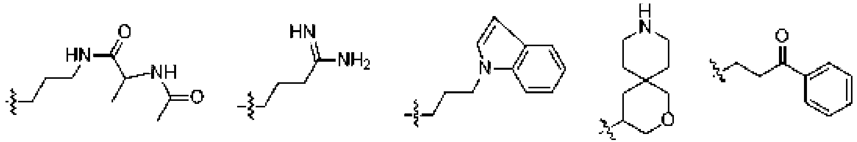

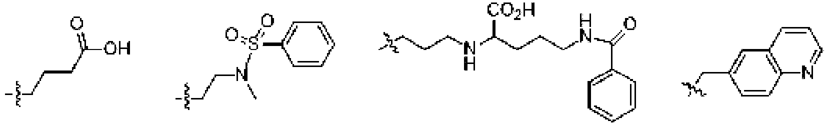

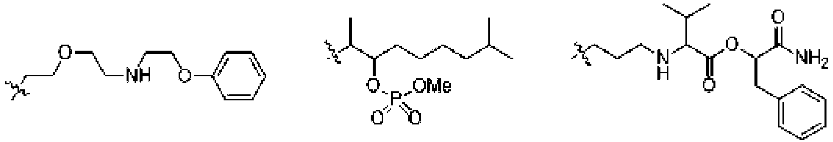

Examples of the substituted or non-substituted alkylacyl are as follows.

### Examples of Non-substituted Alkylacyl Radical

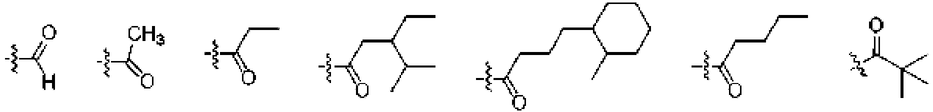

### Examples of Substituted Alkylacyl Radical

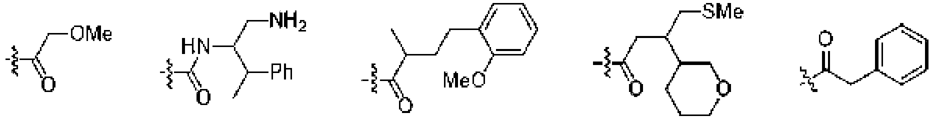

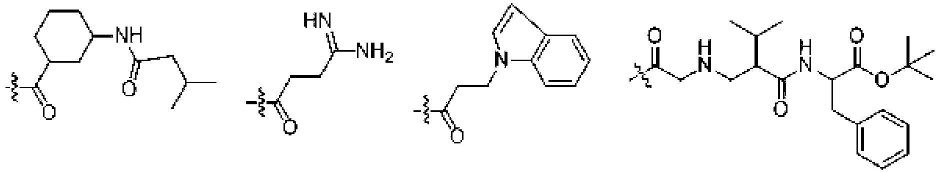

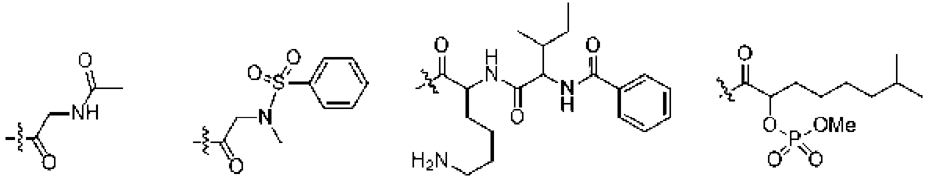

Examples of the substituted or non-substituted arylacyl are as follows.

### Examples of Substituted or Non-substituted Arylacyl Radical

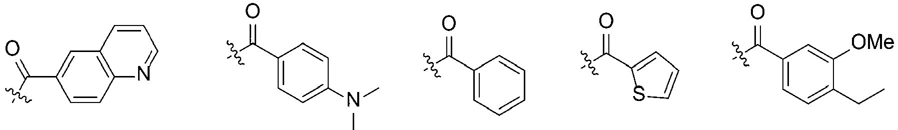

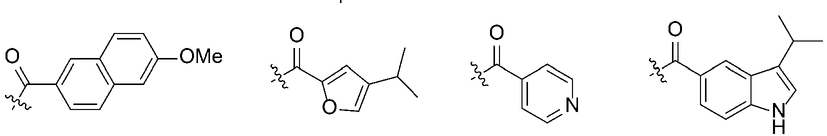

Examples of the substituted or non-substituted alkylamino and substituted or non-substituted arylamino are as follows.

### Examples of Substituted Alkylamino or Arylamino Radical

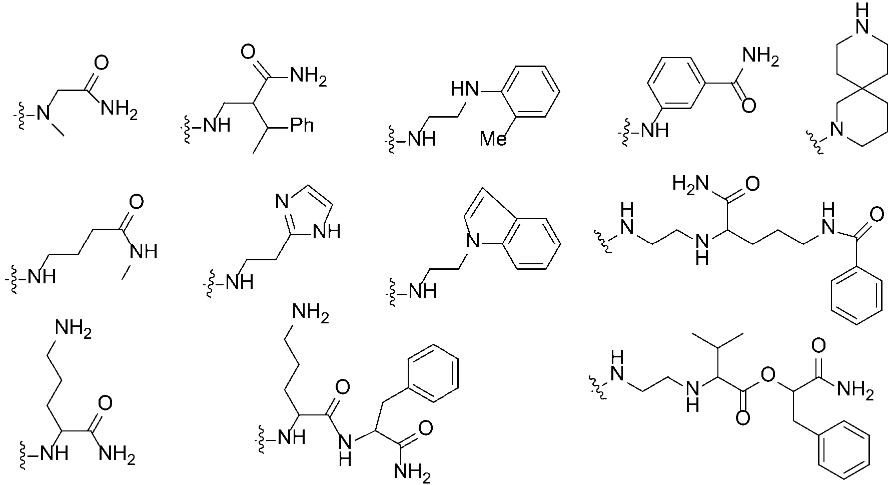

Examples of the substituted or non-substituted aryl are as follows.

### Examples of Substituted or Non-substituted Aryl Radical

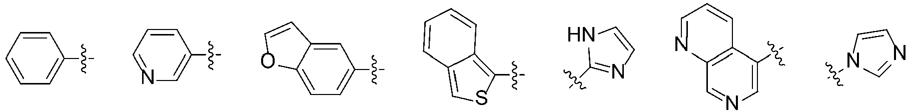

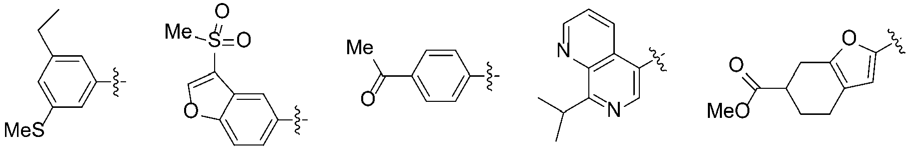

Examples of the substituted or non-substituted alkylsulfonyl and substituted or non-substituted arylsulfonyl are as follows.

### Examples of Substituted or Non-substituted Alkylsulfonyl or Arylsulfonyl Radical

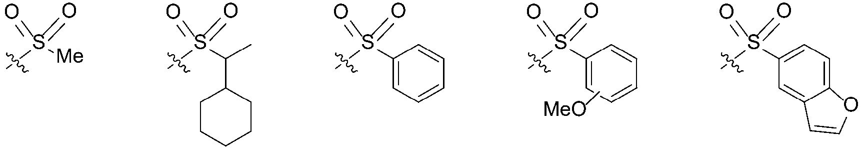

Examples of the substituted or non-substituted alkylphosphonyl and substituted or non-substituted arylphosphonyl are as follows.

### Examples of Substituted or Non-substituted Alkyl- or Aryl-phosphonyl Radical

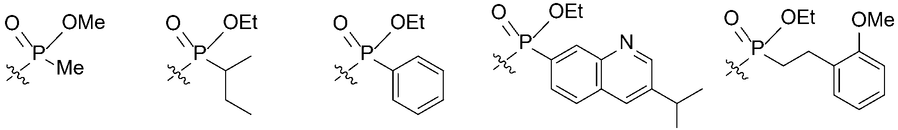

Examples of the substituted or non-substituted alkyloxycarbonyl are as follows.

### Examples of Substituted or Non-substituted Alkyloxycarbonyl Radical

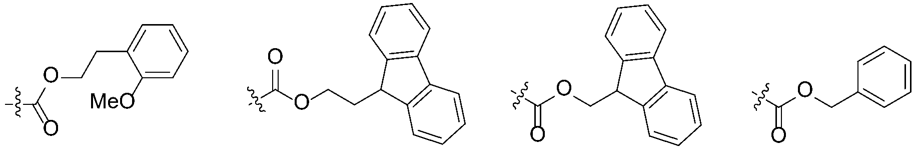

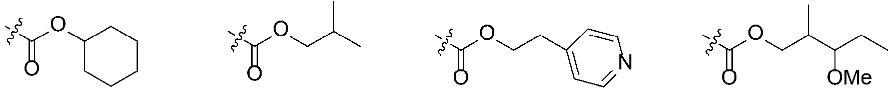

Examples of the substituted or non-substituted aryloxycarbonyl are as follows.

### Examples of Substituted or Non-substituted Aryloxycarbonyl Radical

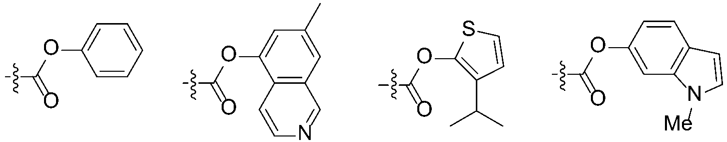

Examples of the substituted or non-substituted alkylaminocarbonyl are as follows.

### Examples of Substituted or Non-substituted Alkylaminocarbonyl Radical

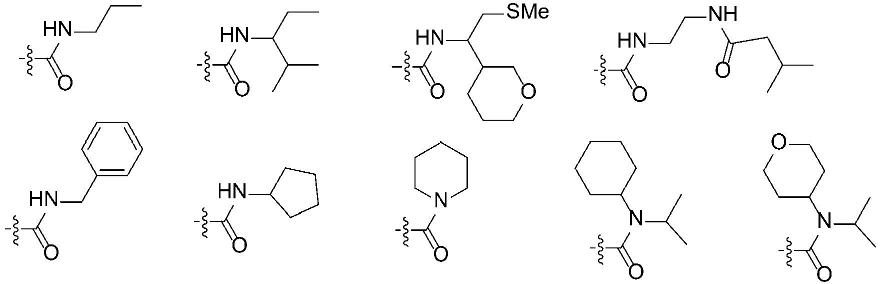

Examples of the substituted or non-substituted arylaminocarbonyl are as follows.

### Examples of Substituted or Non-substituted Arylaminocarbonyl Radical

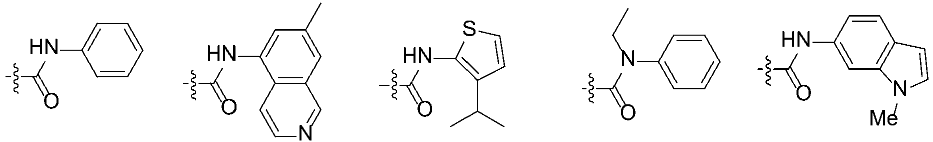

In addition, the N- and C-termini of Chemical Formula 1 may include the following substituents.

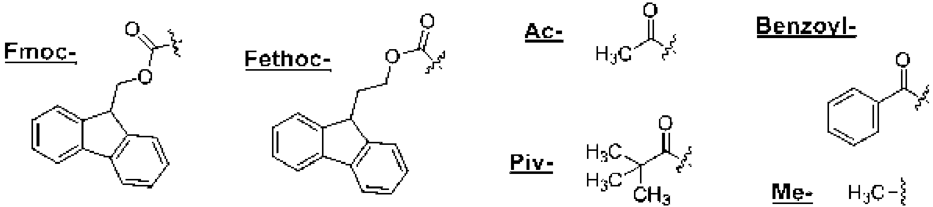

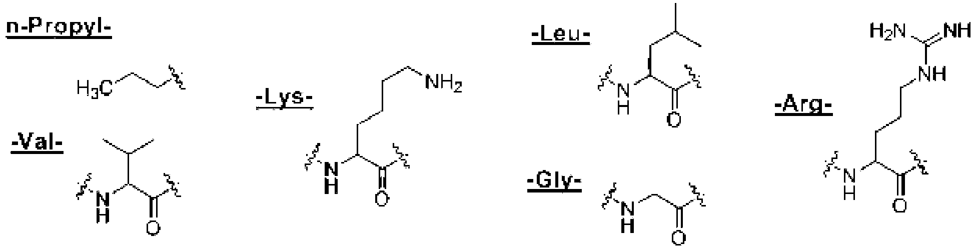

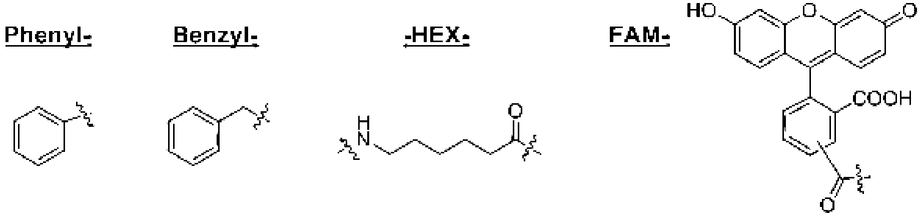

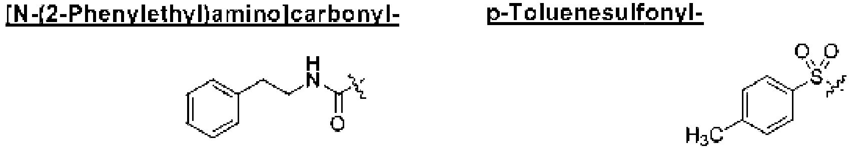

### H- Hydrido (H) -NH₂ non-substituted amino

Abbreviations for substituents at the N-terminus and C-terminus are specifically described as follows: "Fmoc-" is an abbreviation for "[(9-fluorenyl)methyloxy]carbonyl]-"; "Fethoc-" is an abbreviation for "[2-(9-fluorenyl)ethyl-1-oxy]carbonyl"; "Ac-" is an abbreviation for "acetyl-"; "benzoyl-" is an abbreviation for "benzenecarbonyl-"; "Piv-" is an abbreviation for "pivalyl-"; "Me-" is an abbreviation for "methyl-"; "n-Propyl-" is an abbreviation for 1-(n-propyl)-"; "H-" is an abbreviation for a "hydrido-" group; "p-Toluenesulfonyl" is an abbreviation for "(4-methylbenzene)-1-sulfonyl-"; "-Lys-" is an abbreviation for an amino acid residue "lysine"; "-Val-" is an abbreviation for an amino acid residue "valine"; "-Leu-" is an abbreviation for an amino acid residue "leucine"; "-Arg-" is an abbreviation for an amino acid residue "arginine"; "-Gly-" is an abbreviation for an amino acid residue "glycine"; "[N-(2-Phenylethyl)amino]carbonyl-" is an abbreviation for "[N-1-(2-phenylethyl)amino]carbonyl-"; "Benzyl-" is an abbreviation for "1-(phenyl)methyl-"; "Phenyl-" is an abbreviation for "phenyl-"; "Me-" is an abbreviation for "methyl-"; "-HEX-" is an abbreviation for "6-amino-1-hexanoyl-", "FAM-" is an abbreviation for "5, or 6-fluoresceincarbonyl- (mixture of isomers)"; "-NH2" is an abbreviation for a non-substituted "-amino" group.

Examples of substituents at the N-terminus and C-terminus in the compound of Chemical Formula 1 in the present invention are merely intended to show the diversity of permissible substituents, and do not limit the scope of the present invention. In particular, the sequence of the PNA oligomer will be more important than the substituents at the N-terminus and/or C-terminus of the PNA oligomer for binding to the target RNA.

Furthermore, in Chemical Formula 1, B₁, B₂, ......, Bₙ₋₁ and Bₙ are each independently selected from a natural nucleobase and an unnatural nucleobase of adenine (A), guanine (G), thymine (T), or cytosine (C), and one or more of B₁ to Bₙ includes a modified nucleobase.

The present inventors constructed a 20-mer PNA oligomer complementary to the target region based on the HCoV-OC43 RNA sequence provided by NCBI (NCBI Reference Sequence: NC_006213) (Table 1).

**[Table 1]**

| PNA oligomer | PNA sequence (N → C) | Target region |
|---|---|---|
| PNA 1 | Fethoc-GCA(5)-CGC-AA(5)A-TCG(6)-CTC-ACA(5)-AT-NH2 | 5' UTR |
| PNA 2 | Fethoc-TGA-A(5)GC-GGG(6)-ATG-CA(5)C-GCA(5)-CG-NH2 | 5' UTR |
| PNA 3 | Fethoc-TCA-GTG-AA(5)G-CGG(6)-GAT-GCA(5)-CGC- A(5)CG-NH2 | 5' UTR |
| PNA 4 | Fethoc-TCA(5)-GTG-AA(5)G-CGG(6)-GAT-GCA(5)-CGC- A(5)CG-NH2 | 5' UTR |
| PNA 5 | Fethoc-CTA(5)-ACA-A(5)GA-GAT-CA(5)G-TGA-A(5)G-NH2 | 5' UTR |
| PNA 6 | Fethoc-AGA(5)-TCT-A(5)AC-AA(5)G-AGA-TCA(5)-GTG- AA(5)G-NH2 | 5' UTR |
| PNA 7 | Fethoc-C(1O2)TA-ACA-A(5)GA-GAT-CA(5)G-TGA-A(5)G- NH2 | 5' UTR |
| PNA 8 | Fethoc-CTA(5)-ACA-A(5)GA-GAT-CA(5)G-TGA-AG(6)-NH2 | 5' UTR |
| PNA 9 | Fethoc-AGA(5)-TTA-CAA(5)-AAA-GA(5)T-CTA-A(5)C-NH2 | 5' UTR |
| PNA 10 | Fethoc-GTT-TA(5)G-ATT-A(5)CA-AA(5)A-AGA(5)-TCT- AA(5)C-NH2 | 5' UTR |
| PNA 11 | Fethoc-GTT-TA(5)G-ATT-A(5)CA-AA(5)A-AGA(5)-TC-NH2 | 5' UTR |
| PNA 12 | Fethoc-GTT-TA(5)G-ATT-A(5)CA-AA(5)A-AGA-TC(1O2)- NH2 | 5' UTR |
| PNA 13 | Fethoc-G(6)TT-TAG-A(5)TT-ACA-AA(5)A-AGA(5)-TC-NH2 | 5' UTR |
| PNA 14 | Fethoc-GTT-TA(5)G-ATT-A(5)CA-AA(5)A-AGA-TC-NH2 | 5' UTR |
| PNA 15 | Fethoc-G(6)TT-TTT-A(5)TA-AA(5)G-TTT-A(5)GA-TT-NH2 | 5' UTR |
| PNA 16 | Fethoc-TTA(5)-CA(5)G-GGA(5)-GTG-GA(5)T-GTT-TT-NH2 | 5' UTR |
| PNA 17 | Fethoc-G(6)CC-CAC-A(5)AG-CAT-A(5)GA-TTA(5)-CA-NH2 | 5' UTR |
| PNA 18 | Fethoc-A(5)CA-TC(1O2)T-G(6)AA-TC(1O2)A-A(5)-NH2 | Nsp3 |
| PNA 19 | Fethoc-TTG-A(5)TT-C(1O2)AG-A(5)TG(6)-T-NH2 | Nsp3 |
| PNA 20 | Fethoc-AG(6)T-TA(5)T-TA(5)C-A(5)AC(1O2)-T-NH2 | Nsp3 |
| PNA 21 | Fethoc-AG(6)T-TG(6)T-AA(5)T-AA(5)C-T-NH2 | Nsp3 |
| PNA 22 | Fethoc-TTT-TTA(5)-AAC-G(6)GG-TTC-G(6)GG-G(6)T-NH2 | Nsp12 |
| PNA 23 | Fethoc-A(5)CC-CCG-A(5)AC-CCG(6)-TTT-AAA(5)-AA-NH2 | Nsp12 |
| PNA 24 | Fethoc-A(5)CC-CCG-A(5)AC-CCG(6)-TTT-A(5)AA-A(5)A- NH2 | Nsp12 |
| PNA 25 | Fethoc-A(5)CC-C(1O2)CG-AA(5)C-CCG(6)-TTT-A(5)AA- A(5)A-NH2 | Nsp12 |
| PNA 26 | Fethoc-TTT-TTA(5)-AAC-G(6)GG-TTC-G(6)GG-G(6)TA- CGA-NH2 | Nsp12 |
| PNA 27 | Fethoc-TCG-TΛ(5)C-CCC-GΛ(5)Λ-CCC-G(6)TT-TΛΛ- A(5)AA-NH2 | Nsp12 |
| PNA 28 | Fethoc-TCG-TA(5)C-CC(1O2)C-GAA(5)-CCC-G(6)TT- TA(5)A-AA(5)A-NH2 | Nsp12 |
| PNA 29 | Fethoc-TTT-TTA(5)-AAC(1O2)-GGG(6)-TTC-G(6)GG-G(6)T- NH2 | Nsp12 |
| PNA 30 | Fethoc-TTT-TTA(5)-AAC-G(6)GG-TTC(1O2)-GGG-G(6)T- NH2 | Nsp12 |
| PNA 31 | Fethoc-A(5)AC(1O2)-CCG(6)-TTT-A(5)AA-A(5)A-NH2 | Nsp12 |
| PNA 32 | Fethoc-TTT-TTA(5)-AA(5)C-G(6)GG(6)-TT-NH2 | Nsp12 |
| PNA 33 | Fethoc-TTA(5)-AAC-G(6)GG-TTC-G(6)GG-G(6)T-NH2 | Nsp12 |
| PNA 34 | Fethoc-A(5)TA-G(6)CA-GC(1O2)A-TTA(5)-CCA(5)- TCC(1O2)-TG-NH2 | Nsp12 |
| PNA 35 | Fethoc-C(1O2)AG-G(6)AT-G(6)GT-A(5)AT-GC(1O2)T-GCT- A(5)T-NH2 | Nsp12 |
| PNA 36 | Fethoc-G(6)GA-TA(5)A-TC(1O2)C-CA(5)A-CC(1O2)C- ATA(5)-AG-NH2 | Nsp12 |
| PNA 37 | Fethoc-C(1O2)TT-A(5)TG-G(6)GT-TG(6)G-GA(5)T-TAT- C(1O2)C-NH2 | Nsp12 |
| PNA 38 | Fethoc-G(6)TA-A(5)TA-G(6)CA-A(5)CA(5)-TT-NH2 | Nsp14 |
| PNA 39 | Fethoc-A(5)AT-G(6)TT-G(6)CT-A(5)TT-A(5)C-NH2 | Nsp14 |
| PNA 40 | Fethoc-CA(5)C-TC(1O2)T-AG(6)T-G(6)TC-AA(5)A- TC(1O2)T-AC-NH2 | Nsp14 |
| PNA 41 | Fethoc-G(6)TA-GA(5)T-TTG(6)-AC(1O2)A-CTA(5)-GAG(6)- TG-NH2 | Nsp14 |
| PNA 42 | Fethoc-G(6)TG-TG(6)G-AA(5)T-GC(1O2)A-TG(6)T-TTA(5)- TT-NH2 | Nsp15 |
| PNA 43 | Fethoc-A(5)AT-A(5)AA-C(1O2)AT-G(6)CA-TTC(1O2)-CAC- A(5)C-NH2 | Nsp15 |
| PNA 44 | Fethoc-ATC-A(5)CA-AGA(5)-TGC(1O2)-AA(5)T-TTA(5)- GG(6)T-GG(6)T-GC-NH2 | Nsp15 |
| PNA 45 | Fethoc-ATC-A(5)CA-AGA(5)-TGC-AA(5)T-TTA(5)-GGT- G(6)GT-GC-NH2 | Nsp15 |
| PNA 46 | Fethoc-CA(5)C-AAG-A(5)TG-C(1O2)AA(5)-TTT-A(5)GG(6)- TGG(6)-TGC-NH2 | Nsp15 |
| PNA 47 | Fethoc-CAA-GA(5)T-GC(1O2)A-A(5)TT-TA(5)G-G(6)TG- G(6)TG-C-NH2 | Nsp15 |
| PNA 48 | Fethoc-AGA(5)-TGC(1O2)-AA(5)T-TTA(5)-GG(6)T-GG(6)T- GC-NH2 | Nsp15 |
| PNA 49 | Fethoc-AGA-TGC(1O2)-AA(5)T-TTA(5)-GG(6)T-GG(6)T-GC- NH2 | Nsp15 |
| PNA 50 | Fethoc-AGA-TGC(1O2)-AA(5)T-TTA(5)-GGT-GG(6)T-GC- NH2 | Nsp15 |
| PNA 51 | Fethoc-TGC(1O2)-AA(5)T-TTA(5)-GG(6)T-GG(6)T-GCA(5)- GT-NH2 | Nsp15 |
| PNA 52 | Fethoc-TGC(1O2)-AA(5) T-TTA(5)-GG(6)-NH2 | Nsp15 |
| PNA 53 | Fethoc-TGC(1O2)-AA(5)T-TTA(5)-GG(6)T-GG(6)T-GC-NH2 | Nsp15 |
| PNA 54 | Fethoc-TGC-AAT-TTA-GGT-GGT-GC-NH2 | Nsp15 |
| PNA 55 | Fethoc-TGC-AA(5)T-TTA(5)-GG(6)T-GG(6)T-GC-NH2 | Nsp15 |
| PNA 56 | Fethoc-TG(6)C-AA(5)T-TTA(5)-GG(6)T-GG(6)T-GC-NH2 | Nsp15 |
| PNA 57 | Fethoc-TG(6)C-AA(5)T-TTA-G(6)GT-G(6)GT-G(6)C-NH2 | Nsp15 |
| PNA 58 | Fethoc-TGC-AA(5)T-TTA-GGT-GGT-GC-NH2 | Nsp15 |
| PNA 59 | Fethoc-TGC-AAT-TTA(5)-GGT-GGT-GC-NH2 | Nsp15 |
| PNA 60 | Fethoc-TGC-AA(5)T-TTA(5)-GGT-GGT-GC-NH2 | Nsp15 |
| PNA 61 | Acetyl-TGC-AAT-TTA-GGT-GGT-GC-NH2 | Nsp15 |
| PNA 62 | Acetyl-TGC-AA(5)T-TTA-GGT-GGT-GC-NH2 | Nsp15 |
| PNA 63 | Acetyl-TGC-AAT-TTA(5)-GGT-GGT-GC-NH2 | Nsp15 |
| PNA 64 | Acetyl-TGC-AA(5)T-TTA(5)-GGT-GGT-GC-NH2 | Nsp15 |
| PNA 65 | Acetyl-TGC(1O2)-AA(5)T-TTA(5)-GG(6)T-GG(6)T-GC-NH2 | Nsp15 |
| PNA 66 | H-TGC(1O2)-AA(5)T-TTA(5)-GG(6)T-GG(6)T-GC-NH2 | Nsp15 |
| PNA 67 | Benzoyl-TGC(1O2)-AA(5)T-TTA(5)-GG(6)T-GG(6)T-GC-NH2 | Nsp15 |
| PNA 68 | Fethoc-TGC(1O2)-AA(5)T-TTA(5)-GGT-GG(6)T-GC-NH2 | Nsp15 |
| PNA 69 | Fethoc-GCA(5)-CCA(5)-CC(1O2)T-AAA(5)-TTG(6)-CA-NH2 | Nsp15 |
| PNA 70 | Fethoc-GCA-CCA(5)-CCT-A(5)AA-TTG-CA-NH2 | Nsp15 |
| PNA 71 | Fethoc-GCA(5)-CCA(5)-CCT-A(5)AA-TTG-CA-NH2 | Nsp15 |
| PNA 72 | Fethoc-C(1O2)AA(5)-TTT-A(5)GG(6)-TGG(6)-TG-NH2 | Nsp15 |
| PNA 73 | Fethoc-TTA(5)-CAC(1O2)-CAA(5)-AGC(1O2)-ATA(5)-AA- NH2 | Nsp16 |
| PNA 74 | Fethoc-TTT-A(5)TG-C(I 02)TT-TG(6)G-TG(6)T-AA(5)-NH2 | Nsp16 |

In addition, the present inventors constructed a 20-mer PNA oligomer complementary to the target region based on the SARS-CoV-2 RNA sequence provided by NCBI (NCBI Reference Sequence: NC_045512) (Table 2).

**[Table 2]**

| PNA oligomer | PNA sequence (N → C) | Target region |
|---|---|---|
| PNA 75 | Fethoc-GG(6)T-ATA(5)-AAC(1O2)-CTT-TA(5)A-T-NH2 | 5' UTR |
| PNA 76 | Fethoc-GGA(5)-AGG-TA(5)T-AAA(5)-CCT-TTA(5)-AT-NH2 | 5' UTR |
| PNA 77 | Fethoc-TG(6)T-TAC(1O2)-CTG-G(6)GA-A(5)GG-T-NH2 | 5' UTR |
| PNA 78 | Fethoc-A(5)CC-TTC(1O2)-CCA-G(6)GT-AA(5)C-A-NH2 | 5' UTR |
| PNA 79 | Fethoc-TTG-G(6)TT-TG(6)T-TAC-CTG(6)-GGA-A(5)G-NH2 | 5' UTR |
| PNA 80 | Benzoyl-TTG-G(6)TT-TG(6)T-TAC-CTG(6)-GGA-A(5)G- NH2 | 5' UTR |
| PNA 81 | Fethoc-TTG-G(6)TT-GG(6)T-TTG-TTA(5)-CCT-G(6)G-NH2 | 5' UTR |
| PNA 82 | Fethoc-CG(6)A-AA(5)G-TTG(6)-GTT-G(6)GT-T-NH2 | 5' UTR |
| PNA 83 | Fethoc-AA(5)C-CAA(5)-CCA-A(5)CT-TTC-G(6)-NH2 | 5' UTR |
| PNA 84 | Fethoc-CTA(5)-CAA-GA(5)G-ATC-GA(5)A-AGT-TG(6)- NH2 | 5' UTR |
| PNA 85 | Fethoc-ACA(5)-GAT-CTA(5)-CAA-GA(5)G-ATC-GA(5)- NH2 | 5' UTR |
| PNA 86 | Fethoc-ACA-GA(5)T-CTA-CAA-GA(5)G-ATC-GA-NH2 | 5' UTR |
| PNA 87 | Fethoc-CTA(5)-CAA(5)-GAG-A(5)TC-G-NH2 | 5' UTR |
| PNA 88 | Fethoc-CA(5)G-ATC(1O2)-TAC-A(5)AG-AGA(5)-T-NH2 | 5' UTR |
| PNA 89 | Fethoc-ATC(1O2)-TCT-TG(6)T-AGA(5)-TCT-G(6)-NH2 | 5' UTR |
| PNA 90 | Fethoc-G(6)TT-CG(6)T-TTA(5)-GAG(6)-AAC(1O2)-AGA(5)- TC-NH2 | 5' UTR |
| PNA 91 | Fethoc-GTT-CG(6)T-TTA(5)-GAG-AA(5)C-AGA(5)-TC-NH2 | 5' UTR |
| PNA 92 | Fethoc-GTT-C(1O2)GT-TTA(5)-GAG(6)-AAC-A(5)-NH2 | 5' UTR |
| PNA 93 | Fethoc-TG(6)T-TC(1O2)T-CTA-A(5)AC-GAA(5)-C-NH2 | 5' UTR |
| PNA 94 | Fethoc-GTT-C(1O2)GT-TTA(5)-GAG(6)-AA(5)-NH2 | 5' UTR |
| PNA 95 | Fethoc-GTT-C(1O2)GT-TTA(5)-GAG(6)-NH2 | 5' UTR |
| PNA 96 | Fethoc-GTG(6)-ACA(5)-GCC-A(5)CA-CAG(6)-A-NH2 | 5' UTR |
| PNA 97 | Fethoc-CA(5)G-TGG-CA(5)A-GAT-AA(5)C-A-NH2 | Nsp3 |
| PNA 98 | Fethoc-CA(5)G-TGG(6)-CAA-G(6)AT-AA(5)C-A-NH2 | Nsp3 |
| PNA 99 | Fethoc-CA(5)G-TGG-CA(5)A-GAT-A(5)-NH2 | Nsp3 |
| PNA 100 | Fethoc-TA(5)T-ATG(6)-TTT-A(5)TA-GTG(6)-ACC-A(5)C- NH2 | Nsp3 |
| PNA 101 | Fethoc-TA(5)T-ATG-TTT-A(5)TA-GTG-ACC-A(5)C-NH2 | Nsp3 |
| PNA 102 | Fethoc-TAT-A(5)TG-TTT-ATA-GTG-A(5)CC-AC-NH2 | Nsp3 |
| PNA 103 | Fethoc-A(5)TG-TTT-A(5)TA-GTG-A(5)-NH2 | Nsp3 |
| PNA 104 | Fethoc-TA(5)C-ATT-C(1O2)TA-A(5)CC-A(5)TA-NH2 | Nsp3 |
| PNA 105 | Fethoc-C(1O2)TC-TA(5)T-TA(5)C-G(6)TT-T-NH2 | Nsp3 |
| PNA 106 | Fethoc-CTC-TA(5)T-TA(5)C-GTT-T-NH2 | Nsp3 |
| PNA 107 | Fethoc-CTT-G(6)TT-GC(1O2)T-CTA(5)-TTA-CG(6)T-TT- NH2 | Nsp3 |
| PNA 108 | Fethoc-TTA(5)-CCT-C(1O2)CA(5)-TTA(5)-G-NH2 | Nsp3 |
| PNA 109 | Fethoc-GC(1O2)A-GCA(5)-CTA(5)-CGT-A(5)-NH2 | Nsp3 |
| PNA 110 | Fethoc-TA(5)A-AA(5)A-CTC(1O2)-TAG(6)-GTA(5)-A-NH2 | Nsp4 |
| PNA 111 | Fethoc-ATC-A(5)CA-TG(6)T-CTT-G(6)GA-CA(5)G-TA-NH2 | Nsp5 |
| PNA 112 | Fethoc-CAT-G(6)TC-TTG(6)-GAC-A(5)-NH2 | Nsp5 |
| PNA 113 | Fethoc-CTA(5)-CCA(5)-CA(5)T-GAA(5)-CC-NH2 | Nsp5 |
| PNA 114 | Fethoc-CCA(5)-ACA-CTA(5)-CCA-CA(5)T-GAA(5)-CC-NH2 | Nsp5 |
| PNA 115 | Fethoc-CCA-ACA(5)-CTA-CCA-CA(5)T-GAA-CC-NH2 | Nsp5 |
| PNA 116 | Fethoc-TA(5)C-AA(5)C-CA(5)A-GC(1O2)T-A-NH2 | Nsp5 |
| PNA 117 | Fethoc-CA(5)A-AC(1O2)C-CG(6)T-TTA(5)-AAA(5)-AC-NH2 | Nsp12 |
| PNA 118 | Fethoc-CA(5)A-AA(5)C-CA(5)T-TTC(1O2)-AAG(6)-AC-NH2 | Nsp12 |
| PNA 119 | Fethoc-TTT-TTA(5)-AAC-G(6)GG-TTT-G(6)CG-G(6)T-NH2 | Nsp12 |
| PNA 120 | Fethoc-A(5)CC-GCA-A(5)AC-CCG(6)-TTT-AAA(5)-AA- NH2 | Nsp12 |
| PNA 121 | Fethoc-A(5)CC-GC(1O2)A-AA(5)C-CCG(6)-TTT-AAA(5)- AA-NH2 | Nsp12 |
| PNA 122 | Benzoyl-A(5)CC-GCA-A(5)AC-CCG(6)-TTT-AAA(5)-AA- NH2 | Nsp12 |
| PNA 123 | Fethoc-A(5)GC-C(1O2)CT-G(6)TA-TA(5)C-GA(5)C- ATC(1O2)-AG-NH2 | Nsp12 |
| PNA 124 | Fethoc-A(5)GC-C(1O2)CT-A(5)TA-TA(5)C-GA(5)C- ATC(1O2)-AG-NH2 | Nsp12 |
| PNA 125 | Fethoc-A(5)GC-A(5)CT-A(5)TA-TG(6)C-GC(1O2)C- ATC(1O2)-AG-NH2 | Nsp12 |
| PNA 126 | Benzoyl-A(5)GC-C(1O2)CT-G(6)TA-TA(5)C-GA(5)C- ATC(1O2)-AG-NH2 | Nsp12 |
| PNA 127 | Fethoc-GA(5)A-TA(5)A-TA(5)A-GA(5)A-TC(1O2)T-A-NH2 | Nsp12 |
| PNA 128 | Fethoc-TTA(5)-AAA(5)-CTA-AG(6)T-CTA-G(6)AG-CT-NH2 | Nsp12 |
| PNA 129 | Fethoc-A(5)GC-TCT-A(5)GA-CTT-A(5)GT-TTT-A(5)A-NH2 | Nsp12 |
| PNA 130 | Fethoc-TA(5)A-AA(5)C-TA(5)A-GTC(1O2)-TAG-A(5)-NH2 | Nsp12 |
| PNA 131 | Fethoc-TC(1O2)T-A(5)GA(5)-CTT-AG(6)T-TTT-A(5)-NH2 | Nsp12 |
| PNA 132 | Fethoc-TA(5)A-AA(5)C-TAA(5)-GTC-T-NH2 | Nsp12 |
| PNA 133 | Fethoc-A(5)GA-CTT-A(5)GT-TTT-A(5)-NH2 | Nsp12 |
| PNA 35^{a} | Fethoc-C(1O2)AG-G(6)AT-G(6)GT-A(5)AT-GC(1O2)T-GCT- A(5)T-NH2 | Nsp12 |
| PNA 134 | Fethoc-A(5)GC-ATT-A(5)CC-A(5)TC-C-NH2 | Nsp12 |
| PNA 135 | Fethoc-GGA(5)-TGG-TA(5)A-TG(6)C-T-NH2 | Nsp12 |
| PNA 136 | Fethoc-TCA(5)-TTG(6)-AAT-C(1O2)AT-AA(5)T-A-NH2 | Nsp12 |
| PNA 137 | Fethoc-CA(5)C-TCA(5)-ATA(5)-CTT-G(6)AG-C(1O2)AC- A(5)C-NH2 | Nsp12 |
| PNA 138 | Fethoc-CCA(5)-CC(1O2)T-GG(6)T-TTA(5)-ACA(5)-TAT- A(5)G-NH2 | Nsp12 |
| PNA 139 | Fethoc-TTA(5)-GCA-TA(5)A-GC(1O2)A-G(6)TT-G(6)TG- G(6)C-NH2 | Nsp12 |
| PNA 140 | Fethoc-TAA-AG(6)A-AC(1O2)T-GA(5)C-TTA(5)-A-NH2 | Nsp12 |
| PNA 141 | Fethoc-TTA(5)-AGT-CA(5)G-TTC(1O2)-TTT-A(5)-NH2 | Nsp12 |
| PNA 142 | Fethoc-ATA(5)-ATA-A(5)AG-AA(5)C-TGA(5)-CTT-A(5)A- NH2 | Nsp12 |
| PNA 143 | Fethoc-TTA(5)-AGT-CA(5)G-TTC(1O2)-TTT-A(5)TT-AT- NH2 | Nsp12 |
| PNA 144 | Fethoc-ATA-ATA-A(5)AG-AAC-TGA(5)-CTT-AA-NH2 | Nsp12 |
| PNA 145 | Fethoc-TTA-AGT-CA(5)G-TTC-TTT-A(5)TT-AT-NH2 | Nsp12 |
| PNA 146 | Fethoc-TAA(5)-AGA-A(5)CT-GA(5)C-T-NH2 | Nsp12 |
| PNA 147 | Fethoc-A(5)GT-CA(5)G-TTC(1O2)-TTT-A(5)-NH2 | Nsp12 |
| PNA 148 | Fethoc-TA(5)C-TAC-A(5)AT-C(1O2)TT-TAA(5)-A-NH2 | Nsp14 |
| PNA 149 | Fethoc-GA(5)C-ATT-G(6)GT-AA(5)C-CCT-A(5)AA-G(6)C- NH2 | Nsp14 |
| PNA 150 | Fethoc-A(5)TT-GGT-A(5)AC-CCT-A(5)AA-GCT-A(5)T-NH2 | Nsp14 |
| PNA 151 | Fethoc-ATT-GGT-A(5)AC-CCT-AAA(5)-GCT-AT-NH2 | Nsp14 |
| PNA 152 | Fethoc-GTA(5)-ACC-CTA(5)-AAG-C-NH2 | Nsp 14 |
| PNA 153 | Fethoc-G(6)TA-A(5)CC-CTA(5)-AAG(6)-C-NH2 | Nsp14 |
| PNA 154 | Fethoc-CG(6)T-TGC(1O2)-AA(5)T-TTA(5)-GG(6)T-GG(6)T- GC-NH2 | Nsp14 |
| PNA 53^{a} | Fethoc-TGC(1O2)-AA(5)T-TTA(5)-GG(6)T-GG(6)T-GC-NH2 | Nsp14 |
| PNA 155 | Fethoc-TGC(1O2)-AA(5)T-TTA(5)-GG(6)T-GG(6)T-GCT- G(6)T-NH2 | Nsp14 |
| PNA 156 | Fethoc-TGC-A(5)AT-TTA(5)-GGT-G(6)GT-G(6)CT-GT-NH2 | Nsp14 |
| PNA 157 | Fethoc-TGC(1O2)-AA(6)T-TTA(6)-GG(5)T-GG(5)T-GC-NH2 | Nsp14 |
| PNA 158 | Fethoc-TG(6)C(102)-A(5)A(5)T-TTA-GGT-GGT-GC-NH2 | Nsp14 |
| PNA 159 | Fethoc-TGC-AAT-TTA(5)-G(6)G(6)T-G(6)GT-GC-NH2 | Nsp14 |
| PNA 160 | Fethoc-TGC-AAT-TTA-GGT-G(6)G(6)T-G(6)C(1O2)-NH2 | Nsp14 |
| PNA 161 | Fethoc-GCA(5)-ATT-TA(5)G-GTG-G-NH2 | Nsp14 |
| PNA 162 | Fethoc-GTC(1O2)-ATA(5)-GAA(5)-CAA(5)-A-NH2 | Nspl5 |
| PNA 163 | Fethoc-GCA(5)-AAA-TA(5)T-ACT-CA(5)A-CTG(6)-TG-NH2 | Nsp16 |
| PNA 164 | Fethoc-GCA-AA(5)A-TAT-ACT-CAA(5)-CTG-TG-NH2 | Nsp16 |
| PNA 165 | Fethoc-CA(5)A-AAT-A(5)TA-CTC-A(5)-NH2 | Nsp16 |
| PNA 166 | Fethoc-GA(5)A-CCA(5)-GCA-C(1O2)CA-A(5)A-NH2 | Nsp16 |
| PNA 167 | Fethoc-A(5)TT-ATT-A(5)GT-GA(5)T-ATG-TA(5)C-GA-NH2 | Nsp16 |
| PNA 168 | Fethoc-ATT-ATT-A(5)GT-GAT-ATG-TA(5)C-GA-NH2 | Nsp16 |
| PNA 169 | Fethoc-GTG-A(5)TA-TGT-A(5)CG-A(5)-NH2 | Nsp16 |
| PNA 170 | Fethoc-TA(5)T-AAA(5)-GAT-A(5)AC-AGA(5)-ACA(5)-TT- NH2 | Nsp16 |
| PNA 171 | Fethoc-TA(5)T-AAA(5)-GAT-A(5)AC-AGA(5)-ACA-TT- NH2 | Nsp16 |
| PNA 172 | Fethoc-TAT-AAA(5)-GAT-AAC-AGA(5)-ACA-TT-NH2 | Nsp16 |
| PNA 173 | Fethoc-AA(5)G-ATA(5)-ACA-GA(5)A-C-NH2 | Nsp16 |
| PNA 174 | Fethoc-TCA(5)-TCA(5)-TCT-GA(5)A-GCA(5)-TTT-TT-NH2 | Nsp16 |
| PNA 175 | Fethoc-TCA-TCA(5)-TCT-GAA-GCA(5)-TTT-TT-NH2 | Nsp16 |
| PNA 176 | Fethoc-CA(5)T-CTG-A(5)AG-CA(5)T-T-NH2 | Nsp16 |
| PNA 177 | Fethoc-ACA(5)-AAC-A(5)TT-G(6)TT-C(1O2)GT-T-NH2 | Nsp16-S protein |
| PNA 178 | Fethoc-CTG(6)-CAC(1O2)-CAA(5)-GTG(6)-ACA(5)-TAG(6)- TG-NH2 | S protein |
| PNA 179 | Fethoc-TG(6)T-GTA(5)-CAA(5)-AAA(5)-CT-NH2 | S protein |
| PNA 180 | Fethoc-GA(5)A-AA(5)A-TTA(5)-AAA(5)-CC-NH2 | S protein |
| PNA 181 | Fethoc-AA(5)C-CTA(5)-TCA-A(5)TT-TG(6)-NH2 | S protein |
| PNA 182 | Fethoc-CTA(5)-ATT-A(5)AT-TG(6)T-TG(6)-NH2 | S protein |
| PNA 183 | Fethoc-CTG(6)-CAG(6)-CTC(1O2)-TAA(5)-TTA-A(5)-NH2 | S protein |
| PNA 184 | Fethoc-CTG(6)-CAG(6)-CTC(1O2)-TAA(5)-NH2 | S protein |
| PNA 185 | Fethoc-CTG(6)-CAG(6)-CTC(1O2)-TAA(5)-TT-NH2 | ORF3a |
| PNA 186 | Fethoc-TG(6)T-GC(1O2)T-TA(5)C-AA(5)A-GG(6)C- AC(1O2)G-CT-NH2 | ORF3a |
| PNA 187 | Fethoc-TG(6)A-GTA(5)-CA(5)T-AA(5)G-TTC(1O2)-GTA(5)- CT-NH2 | E protein |
| PNA 188 | Fethoc-TA(5)C-ATA(5)-AGT-TC(1O2)G-TA(5)-NH2 | E protein |
| PNA 189 | Fethoc-ACA(5)-ATC-G(6)AA-G(6)CG-CA(5)G-TA(5)A- G(6)G-NH2 | E protein |
| PNA 190 | Fethoc-CA(5)A-GA(5)C-TC(1O2)A-CG(6)T-TA(5)A-CA(5)A- TA-NH2 | E protein |
| PNA 191 | Fethoc-A(5)GA-A(5)TT-CA(5)G-A(5)TT-TTT-A(5)AC- A(5)C-NH2 | E protein |
| PNA 192 | Fethoc-TA(5)T-TTA(5)-GTT-C(1O2)GT-TTA(5)-G-NH2 | E protein |
| PNA 193 | Fethoc-CTT-A(5)CT-G(6)TA(5)-CAA(5)-G-NH2 | M protein |
| PNA 194 | Fethoc-AG(6)T-TTG(6)-TTC(1O2)-GTT-TA(5)-NH2 | ORF8 |
| PNA 195 | Fethoc-TCA(5)-TTT-TA(5)C-CG(6)T-CA(5)C-CA(5)C-CA(5)- NH2 | N protein |
| PNA 196 | Fethoc-GTG(6)-CAA(5)-TTT-G(6)CG-G(6)CC-A(5)AT- G(6)T-NH2 | N protein |
| PNA 197 | Fethoc-TG(6)T-CA(5)T-TCT-C(1O2)CT-AA(5)G-A-NH2 | 3' UTR |
| PNA 198 | Fethoc-TC(1O2)T-TAG(6)-GAG-A(5)AT-GA(5)C-A-NH2 | 3' UTR |

In the PNA sequences of Tables 1 and 2 above, X (A, G, T, or C) is each a natural nucleobase, X (p) or X (pOq) is a modified nucleobase, Fethoc- is [2-(9-fluorenyl)ethyl-1-oxy]carbonyl], and the substituent at the N-terminus may be, in addition to Fethoc-, Fmoc- in the structure below [(9-fluorenyl)methyloxy]carbonyl]-, Acetyl (Ac), Benzoyl, or hydrogen.

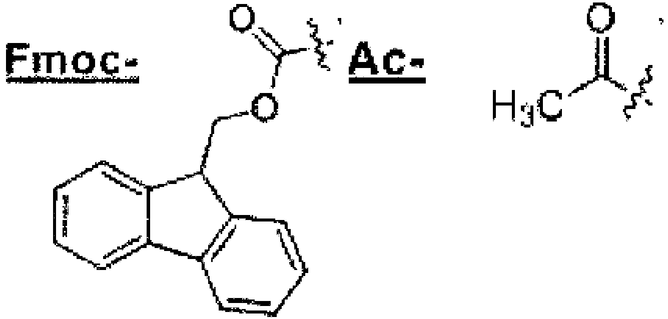

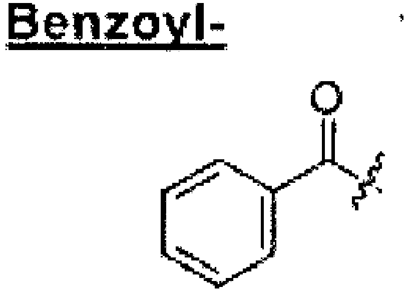

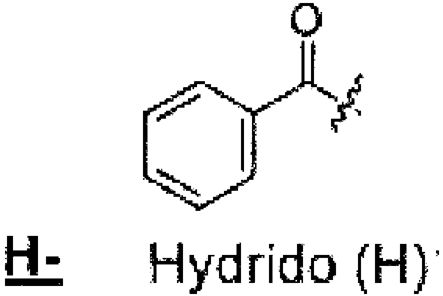

Meanwhile, the PNA derivatives in the following Table 3 target sequences that are conserved across multiple coronaviruses. Specifically, PNA35 targets a sequence common to HCoV-OC43 and SARS-CoV-2, and PNA53 targets a sequence common to HCoV-OC43, SARS-CoV-2, and SARS-CoV.

**[Table 3]**

| | | |
|---|---|---|
| PNA 35 | Fethoc-C(1O2)AG-G(6)AT-G(6)GT-A(5)AT-GC(1O2)T-GCT-A(5)T- NH2 | Nsp12 |
| PNA 53 | Fethoc-TGC(1O2)-AA(5)T-TTA(5)-GG(6)T-GG(6)T-GC-NH2 | Nsp14 |

The present inventors designed and constructed the PNA oligomers of Tables 1 and 2, performed mass spectrometry, and compared the expected mass values of the PNA oligomers during the design with the mass values of the constructed PNAs, thereby confirming that the designed PNA oligomers were constructed (see Examples 2-1 and 3-1).

Next, the present inventors confirmed whether the constructed PNA oligomers could inhibit viral proliferation. First, to confirm whether the constructed HCoV-OC43 RNA-targeting PNA oligomer could bind to viral genomic RNA and inhibit its replication and translation with high efficiency, qRT-PCR was performed to confirm the levels of viral genes in a virus culture solution treated with the PNA oligomer. In this case, it was also confirmed whether an enhanced viral RNA replication inhibition effect could be obtained through a combination of multiple PNA oligomers. For the comparison of the above efficacy, a commercially available drug PF07321332 was used as a control. As a result, it was confirmed that an absolute majority of the constructed PNA oligomers could inhibit the replication of HCoV-OC43 viral RNA, and furthermore, the combination of PNA oligomers exhibited enhanced virus proliferation inhibition activity (see Example 2-2).

Next, it was confirmed whether the constructed SARS-CoV-2 RNA-targeting PNA oligomer could bind to the viral genomic RNA and inhibit its replication with high efficiency. In this case, it was also confirmed whether an enhanced viral RNA replication and translation inhibition effect could be obtained through a combination of multiple PNA oligomers. As a result of the qRT-PCR experiment, it could be confirmed that like the HCoV-OC43-targeting PNA oligomer, the SARS-CoV-2-targeting PNA oligomer effectively inhibited the replication of the target viral gene, and in particular, the combination of multiple PNA oligomers inhibited viral proliferation more effectively. In addition, 20 drugs inhibiting viral proliferation with high efficiency were selected based on qRT-PCR results and subjected to a titer analysis, as a result, it was confirmed that 19 drugs exhibited a high virus proliferation inhibition rate of 90% or more at 1000 nM.

Meanwhile, the present inventors intended to confirm whether the designed and constructed PNA oligomer can be universally used for coronavirus. It was assumed that the modified PNA can maintain binding affinity with the target even when a mismatch is present in the complementary binding region due to its high binding affinity to the target, thereby inhibiting viral proliferation, and as a result of confirming the level of viral protein expression by treating a PNA oligomer having a sequence 100% complementary to a portion of HCoV-OC43 RNA with a SARS-CoV-2 culture solution, it could be confirmed that the HCoV-OC43-targeting PNA oligomer acts even on a SARS-CoV-2 genome including some mismatches and inhibits the protein expression thereof.

From the above, the present inventors have constructed an oligomer capable of complementarily binding to a portion of the coronavirus genome using a modified PNA, which enables the oligomer to penetrate into virus-infected cells without a delivery vehicle and inhibit viral proliferation, and furthermore, since the PNA oligomer can operate even when a mismatch is included in the complementary binding region, the present invention can be provided as a universally usable virus therapeutic agent even when a mutation of the coronavirus occurs.

The compound of Chemical Formula I of the present invention does not require a separate invasive formulation to facilitate systemic delivery for the intended therapeutic or biological activity. In the present specification, the invasive formulation is described as a "delivery vehicle" for enhancing cell permeability. When the compounds of Chemical Formula I are usually dissolved in a phosphate buffer solution (PBS) or saline solution and then administered systemically, the desired therapeutic (analgesic) or biological activity is exhibited in a target tissue (mainly nerve cells). The compound of the present invention does not require a separate strongly invasive formulation for systemic therapeutic activity.

Meanwhile, the *in vivo* administration of oligonucleotides for the treatment of diseases has various difficulties due to the decomposition of enzymes in the blood, mutual binding with substances in the blood, and non-specific delivery to cells. To overcome these difficulties, attempts have been made to improve delivery ability by using nuclease-resistant analogs or linking a structure such as PEG, and this has led to the development of viral vector-based and non-viral delivery systems. However, the viral vector-based delivery system has problems such as immunogenicity caused by the body's immune system, unwanted genome integration, size limitations of a nucleic acid to be loaded, difficulty in repeated administration, the risk of complications, and high production costs. Meanwhile, in the case of non-viral-based delivery systems that encapsulate and deliver nucleic acids such as polymers, lipids, and lipid nanoparticles (LNPs), lipid-based structures using cholesterol or PEG, and polymer-based structures such as PLGA are being used, but these structures have toxicity problems and are limited in that they can only be delivered to specific organs. Further, even in the case of non-natural oligonucleotides used to improve stability, there is a problem in that a delivery vehicle is needed to permeate the cell membrane.

The compound of Chemical Formula I of the present invention does not require a separate delivery vehicle for cell membrane permeation, and thus inhibits the replication of coronavirus within cells even when administered systemically. Therefore, the compound of the present invention is useful for safely treating symptoms associated with coronavirus infectious diseases.

The compound of Chemical Formula I of the present invention can be used together with a pharmaceutically acceptable acid or base, and the pharmaceutically acceptable acid or base includes sodium hydroxide, potassium hydroxide, hydrochloric acid, methanesulfonic acid, citric acid, trifluoroacetic acid, and the like, but is not necessarily limited thereto.

In the present invention, the compound of Chemical Formula I or a pharmaceutically acceptable salt may be administered together with a pharmaceutically acceptable adjuvant, and the adjuvant includes citric acid, hydrochloric acid, tartaric acid, stearic acid, polyethylene glycol, polypropylene glycol, ethanol, isopropanol, sodium bicarbonate, distilled water, a preservative, and the like, but is not limited thereto.

### [Modes of the Invention]

Since the present invention may be modified into various forms and include various exemplary embodiments, specific exemplary embodiments will be illustrated in the drawings and described in detail in the following Detailed Description. However, the description is not intended to limit the present invention to specific exemplary embodiments, and it is to be understood that all the changes, equivalents, and substitutions within the spirit and technical scope of the present invention are included in the present invention. In describing the present invention, when it is determined that the detailed description of the related publicly known art may obscure the gist of the present invention, the detailed description thereof will be omitted.

### [Examples]

### Example 1. General method for synthesizing PNA oligomers

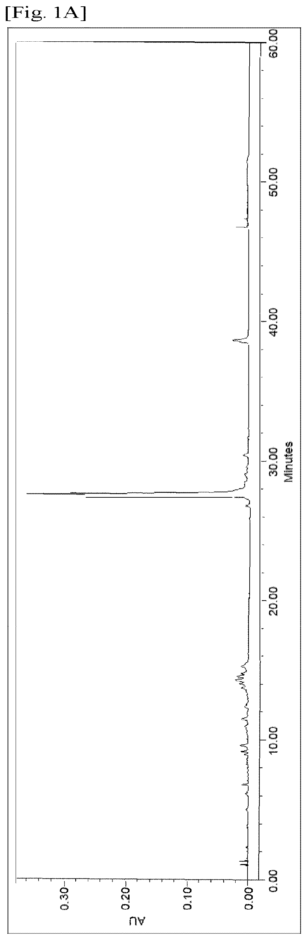

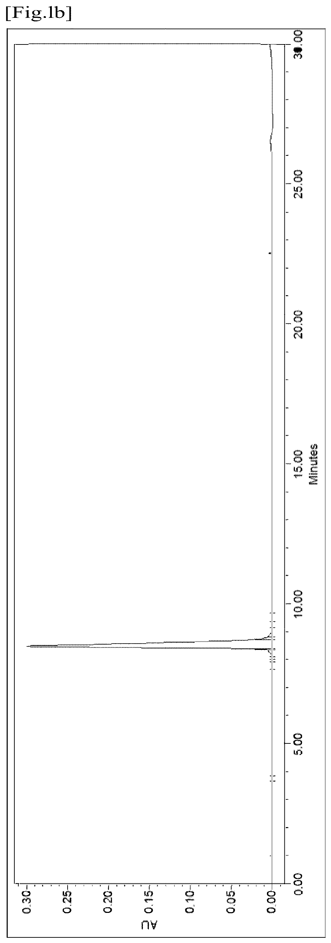

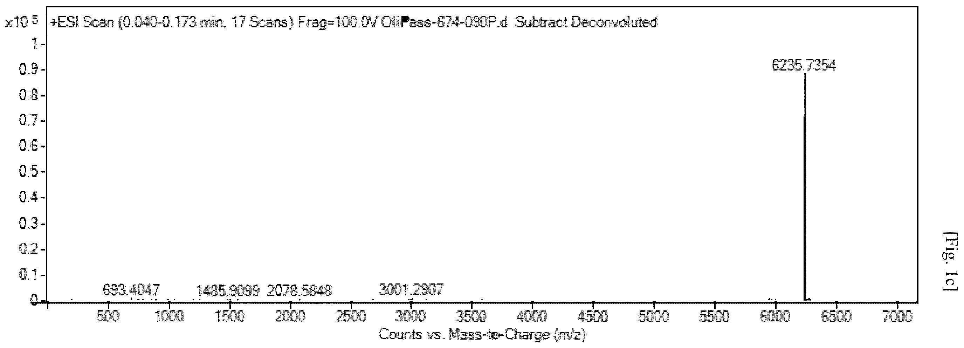

PNA monomers having modified nucleobases were synthesized by the method disclosed in the related art [PCT/KR 2009/001256] or by a slight modification thereof. Using Fmoc[{(9-fluorenyl)methoxy}carbonyl]-protected PNA monomers having modified nucleobases and Fmoc-protected PNA monomers having natural nucleobases, PNA oligomers of Formula (I) were synthesized in the solid phase by the method disclosed in the related art [US6,133,444; WO96/40685] or by a slight modification thereof, as shown in [Reaction Scheme 1]. As the solid phase used for the synthesis, H-Rink Amide-ChemMatrix resin purchased from PCAS BioMatrix Inc. (Quebec, Canada) was usually used. The PNA oligomers thus synthesized were identified by TOF LC/MS and separated and analyzed using C₁₈ -reverse phase UPLC. (Distilled water/acetonitrile or distilled water/methanol, 0.1% TFA) [FIGS. 1A and 1B] are HPLC chromatograms before and after HPLC purification of "PNA123", and [FIG. 1C] is an ESI/TOF/MS spectrum for "PNA123" purified by HPLC, and are provided for the purpose of describing the PNA oligomers of the present invention and are not intended to limit the scope of the present invention.

[Reaction Scheme 1] is a schematic view of a typical process of solid-state synthesis, and each reaction process is briefly provided as follows.

[Activation of H-Rink-ChemMatrix resin] In the case where an Fmoc protecting group is attached to the amine of a resin, a suspension, in which 0.94 mmol (about 200 mg of the resin) and 5 mL of 20% piperidine/dimethylformamide (DMF) are mixed, is vortexed in a libra tube for 5 minutes, and then the DeFmoc solution is filtered and removed. When the resin is washed with 5 mL of methylene chloride (MC), 5 mL of DMF, and 5 mL of DMF for 30 seconds each, the amine of the resin is ready to react with the Fmoc-protected PNA monomer.

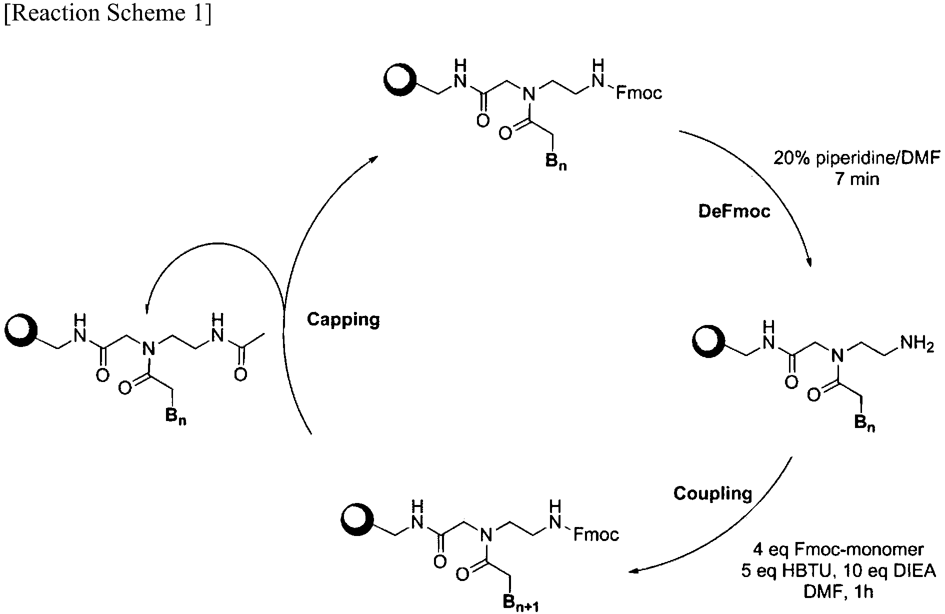

[Fmoc removal (DeFmoc)] In the case where an Fmoc protecting group is attached to the amine of a resin, a suspension, in which 0.94 mmol (about 200 mg of the resin) and 5 mL of 20% piperidine/DMF are mixed, is vortexed in a libra tube for 5 minutes, and then the DeFmoc solution is filtered and removed. When the resin is washed with 5 mL of methylene chloride (MC), 5 mL of DMF, and 5 mL of DMF for 30 seconds each, the amine of the resin is ready to react with the Fmoc-protected PNA monomer.

[Coupling with Fmoc-PNA monomer] The amine of the resin is coupled with the Fmoc-PNA monomer by the following method. 0.235 mmol PNA monomer, 0.235 mmol HBTU, 0.2585 mmol DIEA, and the like are dissolved in 5 mL of anhydrous DMF, and then the solution is added to the resin after 2 minutes. After the resin suspension is vortexed for 1 hour, the solution is filtered and removed, and the resin is washed with 5 mL of MC, 5 mL of DMF, 5 mL of MC, 5 mL of DMF, and 5 mL of DMF for 30 seconds each.

[Capping] After the coupling reaction, an unreacted amine is capped by reacting in 5 mL of a capping solution (a DMF solution of 5% acetic anhydride and 6% 2,6-lutidine) for 5 minutes. After filtering and removing the capping solution, the resin is washed with 5 mL of MC, 5 mL of DMF, and 5 mL of DMF for 30 seconds each.

[Introduction of "Fethoc" group to N-terminus] Under basic coupling conditions, a "Fethoc" group is introduced to the N-terminal amine of the resin by reaction with "Fethoc-OSu", and the chemical structure of "Fethoc-OSu" [CAS No. 179337-69-0, C₂₀H₁₇NO₅, MW 351.36] is as follows.

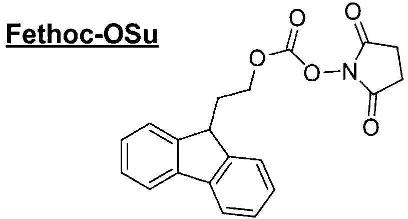

[Separation from resin] PNA oligomers bound to the resin are separated from the resin by reacting in 6 mL of a cleavage solution (a solution of 2% triisopropylsilane and 2% trifluoroacetic acid in water) for 3 hours. The resin is filtered and removed, and the filtrate is concentrated under low pressure. A solid obtained by treating the residue with ether (diethyl ether) is obtained by filtration, and then purified by reverse phase HPLC.

[HPLC analysis and purification] A crude PNA oligomer product separated from the resin is purified by C₁₈-reverse phase HPLC using water/acetonitrile or water/methanol containing 0.1% TFA as the eluent. FIGS. 1A and 1B are examples of Cis-reverse phase UPLC chromatograms before and after purification of "PNA 123."

### Synthetic examples of PNA derivatives of Formula I

PNA oligomers that complementarily bind to the genomic RNA of coronavirus were synthesized by partial modification of the above method. These PNA oligomers targeting the RNA of coronavirus are provided to illustrate the content of the present invention, and are not intended to limit the scope of the present invention.

Tables 1 and 2 above show PNA oligomers targeting HCoV-OC43 RNA or SARS-CoV-2 RNA.

The following Table 4 shows PNA oligomers that are completely complementary to HCoV-OC43 RNA among the constructed PNA oligomers, and Table 5 shows PNA oligomers that are completely complementary to SARS-CoV-2 RNA among the constructed PNA oligomers.

**[Table 4]**

| PNA oligomer | PNA sequence (N → C) | Target region |
|---|---|---|
| PNA 1 | Fethoc-GCA(5)-CGC-AA(5)A-TCG(6)-CTC-ACA(5)-AT-NH2 | 5' UTR |
| PNA 2 | Fethoc-TGA-A(5)GC-GGG(6)-ATG-CA(5)C-GCA(5)-CG-NH2 | 5' UTR |
| PNA 3 | Fethoc-TCΛ-GTG-ΛΛ(5)G-CGG(6)-GΛT-GCΛ(5)-CGC- A(5)CG-NH2 | 5' UTR |
| PNA 4 | Fethoc-TCΛ(5)-GTG-ΛΛ(5)G-CGG(6)-GΛT-GCΛ(5)-CGC- A(5)CG-NH2 | 5' UTR |
| PNA 5 | Fethoc-CTA(5)-ACA-A(5)GA-GAT-CA(5)G-TGA-A(5)G-NH2 | 5' UTR |
| PNA 6 | Fethoc-AGA(5)-TCT-A(5)AC-AA(5)G-AGA-TCA(5)-GTG- AA(5)G-NH2 | 5' UTR |
| PNA 7 | Fethoc-C(1O2)TA-ACA-A(5)GA-GAT-CA(5)G-TGA-A(5)G- NH2 | 5' UTR |
| PNA 8 | Fethoc-CTA(5)-ACA-A(5)GA-GAT-CA(5)G-TGA-AG(6)-NH2 | 5' UTR |
| PNA 9 | Fethoc-AGA(5)-TTA-CAA(5)-AAA-GA(5)T-CTA-A(5)C-NH2 | 5' UTR |
| PNA 10 | Fethoc-GTT-TA(5)G-ATT-A(5)CA-AA(5)A-AGA(5)-TCT- AA(5)C-NH2 | 5' UTR |
| PNA 11 | Fethoc-GTT-TA(5)G-ATT-A(5)CA-AA(5)A-AGA(5)-TC-NH2 | 5' UTR |
| PNA 12 | Fethoc-GTT-TA(5)G-ATT-A(5)CA-AA(5)A-AGA-TC(1O2)-NH2 | 5' UTR |
| PNA 13 | Fethoc-G(6)TT-TAG-A(5)TT-ACA-AA(5)A-AGA(5)-TC-NH2 | 5' UTR |
| PNA 15 | Fethoc-G(6)TT-TTT-A(5)TA-AA(5)G-TTT-A(5)GA-TT-NH2 | 5' UTR |
| PNA 16 | Fethoc-TTA(5)-CA(5)G-GGA(5)-GTG-GA(5)T-GTT-TT-NH2 | 5' UTR |
| PNA 17 | Fethoc-G(6)CC-CAC-A(5)AG-CAT-A(5)GA-TTA(5)-CA-NH2 | 5' UTR |
| PNA 22 | Fethoc-TTT-TTA(5)-AAC-G(6)GG-TTC-G(6)GG-G(6)T-NH2 | Nsp12 |
| PNA 23 | Fethoc-A(5)AC(1O2)-CCG(6)-TTT-A(5)AA-A(5)A-NH2 | Nsp12 |
| PNA 24 | Fethoc-A(5)CC-CCG-A(5)AC-CCG(6)-TTT-A(5)AA-A(5)A-NH2 | Nsp12 |
| PNA 25 | Fethoc-A(5)CC-C(1O2)CG-AA(5)C-CCG(6)-TTT-A(5)AA- A(5)A-NH2 | Nsp12 |
| PNA 26 | Fethoc-TTT-TTA(5)-AAC-G(6)GG-TTC-G(6)GG-G(6)TA-CGA- NH2 | Nsp12 |
| PNA 27 | Fethoc-TCG-TA(5)C-CCC-GA(5)A-CCC-G(6)TT-TAA-A(5)AA- NH2 | Nsp12 |
| PNA 28 | Fethoc-TCG-TA(5)C-CC(1O2)C-GAA(5)-CCC-G(6)TT-TA(5)A- AA(5)A-NH2 | Nsp12 |
| PNA 29 | Fethoc-TTT-TTA(5)-AAC(1O2)-GGG(6)-TTC-G(6)GG-G(6)T- NH2 | Nsp12 |
| PNA 30 | Fethoc-TTT-TTA(5)-AAC-G(6)GG-TTC(1O2)-GGG-G(6)T-NH2 | Nsp12 |
| PNA 33 | Fethoc-TTA(5)-AAC-G(6)GG-TTC-G(6)GG-G(6)T-NH2 | Nsp12 |
| PNA 44 | Fethoc-ATC-A(5)CA-AGA(5)-TGC(1O2)-AA(5)T-TTA(5)- GG(6)T-GG(6)T-GC-NH2 | Nsp15 |
| PNA 45 | Fethoc-ATC-A(5)CA-AGA(5)-TGC-AA(5)T-TTA(5)-GGT- G(6)GT-GC-NH2 | Nsp15 |
| PNA 46 | Fethoc-CA(5)C-AAG-A(5)TG-C(1O2)AA(5)-TTT-A(5)GG(6)- TGG(6)-TGC-NH2 | Nsp15 |
| PNA 47 | Fethoc-CΛΛ-GΛ(5)T-GC(1O2)Λ-Λ(5)TT-TΛ(5)G-G(6)TG- G(6)TG-C-NH2 | Nsp15 |
| PNA 48 | Fethoc-AGA(5)-TGC(1O2)-AA(5)T-TTA(5)-GG(6)T-GG(6)T- GC-NH2 | Nsp15 |
| PNA 49 | Fethoc-AGA-TGC(1O2)-AA(5)T-TTA(5)-GG(6)T-GG(6)T-GC- NH2 | Nsp15 |
| PNA 50 | Fethoc-AGA-TGC(1O2)-AA(5)T-TTA(5)-GGT-GG(6)T-GC-NH2 | Nsp15 |
| PNA 51 | Fethoc-TGC(1O2)-AA(5)T-TTA(5)-GG(6)T-GG(6)T-GCA(5)- GT-NH2 | Nsp15 |
| PNA 52 | Fethoc-TGC(1O2)-AA(5)T-TTA(5)-GG(6)T-GG(6)-NH2 | Nsp15 |
| PNA 53 | Fethoc-TGC(1O2)-AA(5)T-TTA(5)-GG(6)T-GG(6)T-GC-NH2 | Nsp15 |
| PNA 54 | Fethoc-TGC-AAT-TTA-GGT-GGT-GC-NH2 | Nsp15 |
| PNA 55 | Fethoc-TGC-AA(5)T-TTA(5)-GG(6)T-GG(6)T-GC-NH2 | Nsp15 |
| PNA 56 | Fethoc-TG(6)C-AA(5)T-TTA(5)-GG(6)T-GG(6)T-GC-NH2 | Nsp15 |
| PNA 57 | Fethoc-TG(6)C-AA(5)T-TTA-G(6)GT-G(6)GT-G(6)C-NH2 | Nsp15 |
| PNA 58 | Fethoc-TGC-AA(5)T-TTA-GGT-GGT-GC-NH2 | Nsp15 |
| PNA 59 | Fethoc-TGC-AAT-TTA(5)-GGT-GGT-GC-NH2 | Nsp15 |
| PNA 60 | Fethoc-TGC-AA(5)T-TTA(5)-GGT-GGT-GC-NH2 | Nsp15 |
| PNA 61 | Acetyl-TGC-AAT-TTA-GGT-GGT-GC-NH2 | Nsp15 |
| PNA 62 | Acetyl-TGC-AA(5)T-TTA-GGT-GGT-GC-NH2 | Nsp15 |
| PNA 63 | Acetyl-TGC-AAT-TTA(5)-GGT-GGT-GC-NH2 | Nspl5 |
| PNA 64 | Acetyl-TGC-AA(5)T-TTA(5)-GGT-GGT-GC-NH2 | Nsp15 |
| PNA 65 | Acetyl-TGC(1O2)-AA(5)T-TTA(5)-GG(6)T-GG(6)T-GC-NH2 | Nsp15 |
| PNA 66 | H-TGC(1O2)-AA(5)T-TTA(5)-GG(6)T-GG(6)T-GC-NH2 | Nsp15 |
| PNA 68 | Fethoc-TGC(1O2)-AA(5)T-TTA(5)-GGT-GG(6)T-GC-NH2 | Nsp15 |
| PNA 70 | Fethoc-GCA-CCA(5)-CCT-A(5)AA-TTG-CA-NH2 | Nsp15 |
| PNA 71 | Fethoc-GCA(5)-CCA(5)-CCT-A(5)AA-TTG-CA-NH2 | Nsp15 |
| PNA 72 | Fethoc-C(1O2)AA(5)-TTT-A(5)GG(6)-TGG(6)-TG-NH2 | Nsp15 |

**[Table 5]**

| PNA oligomer | PNA sequence (N → C) | Target region |
|---|---|---|
| PNA 35 | Fethoc-C(1O2)ΛG-G(6)ΛT-G(6)GT-Λ(5)ΛT-GC(1O2)T-GCT- A(5)T-NH2 | Nsp12 |
| PNA 53 | Fethoc-TGC(1O2)-AA(5)T-TTA(5)-GG(6)T-GG(6)T-GC-NH2 | Nsp14 |
| PNA 76 | Fethoc-GGA(5)-AGG-TA(5)T-AAA(5)-CCT-TTA(5)-AT-NH2 | 5' UTR |
| PNA 79 | Fethoc-TTG-G(6)TT-TG(6)T-TAC-CTG(6)-GGA-A(5)G-NH2 | 5' UTR |
| PNA 81 | Fethoc-TTG-G(6)TT-GG(6)T-TTG-TTA(5)-CCT-G(6)G-NH2 | 5' UTR |
| PNA 84 | Fethoc-CTA(5)-CAA-GA(5)G-ATC-GA(5)A-AGT-TG(6)-NH2 | 5' UTR |
| PNA 88 | Fethoc-CA(5)G-ATC(1O2)-TAC-A(5)AG-AGA(5)-T-NH2 | 5' UTR |
| PNA 91 | Fethoc-GTT-CG(6)T-TTA(5)-GAG-AA(5)C-AGA(5)-TC-NH2 | 5' UTR |
| PNA 105 | Fethoc-C(1O2)TC-TA(5)T-TA(5)C-G(6)TT-T-NH2 | Nsp3 |
| PNA 111 | Fethoc-ATC-A(5)CA-TG(6)T-CTT-G(6)GA-CA(5)G-TA-NH2 | Nsp5 |
| PNA 120 | Fethoc-A(5)CC-GCA-A(5)AC-CCG(6)-TTT-AAA(5)-AA-NH2 | Nsp 12 |
| PNA 121 | Fethoc-A(5)CC-GC(1O2)A-AA(5)C-CCG(6)-TTT-AAA(5)-AA- NH2 | Nsp12 |
| PNA 123 | Fethoc-A(5)GC-C(1O2)CT-G(6)TA-TA(5)C-GA(5)C-ATC(1O2)- AG-NH2 | Nsp12 |
| PNA 129 | Fethoc-A(5)GC-TCT-A(5)GA-CTT-A(5)GT-TTT-A(5)A-NH2 | Nsp12 |
| PNA 143 | Fethoc-TTA(5)-AGT-CA(5)G-TTC(1O2)-TTT-A(5)TT-AT-NH2 | Nsp12 |
| PNA 157 | Fethoc-TGC(1O2)-AA(6)T-TTA(6)-GG(5)T-GG(5)T-GC-NH2 | Nsp14 |

### Example 2. Analysis of PNA oligomers targeting HCoV-OC43 RNA

### 2-1. Mass structure analysis

The following Table 6 shows the results of mass structure analysis of the constructed PNA oligomers targeting HCoV-OC43.

**[Table 6]**

| PNA oligomer | Exact Mass, m/z | | PNA oligomer | Exact Mass, m/z | |
|---|---|---|---|---|---|
| | theor.^{a} | obs.^{b} | | theor.^{a} | obs.^{b} |
| PNA 1 | 6007.65 | 6007.47 | PNA 45 | 7858.39 | 7858.32 |
| PNA 2 | 6144.66 | 6144.72 | PNA 46 | 7513.34 | 7513.29 |
| PNA 3 | 7228.08 | 7228.05 | PNA 47 | 6887.02 | 6886.98 |
| PNA 4 | 7328.18 | 7328.15 | PNA 48 | 6360.81 | 6360.86 |
| PNA 5 | 6136.68 | 6136.69 | PNA 49 | 6260.71 | 6260.69 |
| PNA 6 | 7344.21 | 7344.15 | PNA 50 | 6161.6 | 6161.64 |
| PNA 7 | 6133.63 | 6133.63 | PNA 51 | 6351.8 | 6351.85 |
| PNA 8 | 6135.68 | 6135.67 | PNA 52 | 4611.06 | 4611.17 |
| PNA 9 | 6079.68 | 6079.56 | PNA 53 | 5419.37 | 5419.41 |
| PNA 10 | 7269.19 | 7269.16 | PNA 54 | 4923.91 | 4923.96 |
| PNA 11 | 6101.66 | 6101.54 | PNA 55 | 5322.32 | 5322.39 |
| PNA 12 | 6098.62 | 6098.6 | PNA 56 | 5421.42 | 5421.49 |
| PNA 13 | 6100.67 | 6100.66 | PNA 57 | 5420.43 | 5420.47 |
| PNA 15 | 2103.63 | 6103.69 | PNA 58 | 5024.01 | 5024.06 |
| PNA 16 | 6171.63 | 6171.74 | PNA 59 | 5024.01 | 5024.07 |
| PNA 17 | 6031.66 | 6031.37 | PNA 60 | 5124.11 | 5124.16 |
| PNA 22 | 6144.64 | 6144.67 | PNA 61 | 4729.84 | 4729.74 |
| PNA 23 | 5991.65 | 5991.73 | PNA 62 | 4829.94 | 4829.97 |
| PNA 24 | 6091.75 | 6091.83 | PNA 63 | 4829.94 | 4829.93 |
| PNA 25 | 6188.81 | 6188.91 | PNA 64 | 4930.04 | 4930.07 |
| PNA 26 | 7237.07 | 7237.03 | PNA 65 | 5225.3 | 5225.25 |
| PNA 27 | 7066.07 | 7066.02 | PNA 66 | 5183.29 | 5183.33 |
| PNA 28 | 7263.22 | 7263.14 | PNA 68 | 5320.27 | 5320.27 |
| PNA 29 | 6241.69 | 6241.62 | PNA 70 | 4991.12 | 4991.14 |
| PNA 30 | 6142.58 | 6142.57 | PNA 71 | 5091.22 | 5091.24 |
| PNA 33 | 5346.33 | 5346.34 | PNA 72 | 4611.06 | 4611.1 |
| PNA 44 | 8054.55 | 8054.52 | | | |

### a) Theoretical value, b) Observed value.

### 2-2. Evaluation of antiviral efficacy of PNA oligomers using qRT-PCR

The efficacy of drugs based on the constructed PNA oligomer was evaluated to determine how well the drugs could block viral gene replication.

Additionally, to confirm whether the antiviral efficacy is enhanced during treatment with a combination of PNA oligomers, an experiment was also performed using a drug (Combi 1) in which PNA 46 and PNA 8 are combined.

In order to analyze the level of viral RNA in viral particles present in a virus culture solution to determine how well the constructed PNA oligomer-based drugs inhibit viral genetic replication, qRT-PCR was performed.

Specifically, HCT8 cells cultured in 6-well plates one day before for drug screening were infected with HCoV-OC43 at a dose of 500 TCID50 using a serum-free RPMI medium. At the same time as infection, the cells were also treated with 1 µM of PNA oligomer, and after 30 minutes, the cells were washed twice with serum-free RPMI, and then the medium was replaced with an RPMI-Complete medium (containing 10% FBS and 1% penicillin/streptomycin) and the cells were treated with 1 µM of PNA oligomer. On Day 3 after infection, viral RNA was extracted from 140 µL of the cell supernatant using a QIAamp viral RNA mini kit manufactured by Qiagen. Virus extraction was performed according to the kit manual. The viral titer of the extracted viral RNA was measured by real-time PCR using a One step TB green kit manufactured by Takara. The primers used are as follows. HCoV-OC43 primer; Nucleoprotein (NP) gene (5'-AGCAACCAGGCTGATGTCAATACC-3', 5'-AGCAGACCTTCCTGAGCCTTCAAT-3'). Real-time PCR conditions are as follows. Temperature Stage 1: Reverse transcription 42°C 5 min, 95°C 10 sec, 1 Cycle. Stage 2: PCR reaction 95°C 5 sec, 60°C 34 sec, 35 Cycles. Stage 3: melting curve analysis 95°C 15 sec, 65°C 60 sec, 95°C 15 sec.

The results of the antiviral efficacy evaluation of PNA oligomers and their combinations targeting HCoV-OC43 through qRT-PCR-based viral RNA level analysis are shown in Table 7 below.

**[Table 7]**

| PNA oligomer | Inhibition% | PNA oligomer | Inhibition% |
|---|---|---|---|
| PNA 1 | 31.78 | PNA 45 | 85.13 |
| PNA 2 | 61.44 | PNA 46 | 73.15 |
| PNA 3 | 64.93 | PNA 47 | 65.28 |
| PNA 4 | 55.2 | PNA 48 | 58.95 |
| PNA 5 | 57.12 | PNA 49 | 66.92 |
| PNA 6 | 60.75 | PNA 50 | 68.55 |
| PNA 7 | 50.13 | PNA 51 | 1.44 |
| PNA 8 | 76.47 | PNA 52 | 28.11 |
| PNA 9 | 3.36 | PNA 53 | 56.68 |
| PNA 10 | 40.44 | PNA 54 | 28.03 |
| PNA 11 | 33.4 | PNA 55 | -2.77 |
| PNA 12 | 43.36 | PNA 56 | 2.02 |
| PNA 13 | 40.94 | PNA 57 | 21.56 |
| PNA 15 | 29.88 | PNA 58 | 31.55 |
| PNA 16 | 44.97 | PNA 59 | 34.93 |
| PNA 17 | 57.69 | PNA 60 | 46.93 |
| PNA 22 | 58.67 | PNA 61 | 11.7 |
| PNA 23 | 56.12 | PNA 62 | 24.45 |
| PNA 24 | 43.51 | PNA 63 | 10.36 |
| PNA 25 | 32.47 | PNA 64 | 46.9 |
| PNA 26 | 41.64 | PNA 65 | 52.75 |
| PNA 27 | 40.12 | PNA 66 | 44.26 |
| PNA 28 | 70.19 | PNA 68 | 73.71 |
| PNA 29 | 55.58 | PNA 70 | 23.06 |
| PNA 30 | 53.76 | PNA 71 | 10.76 |
| PNA 33 | 30.51 | PNA 72 | 22.34 |
| PNA 44 | 71.17 | Combi 1 | 79.55 |
| | | PF07321332^{a} | 97.98 |
| ^{a)} PF07321332 (Nirmatrelvir, viral protease inhibitor developed by Pfizer) | | | |

### Example 3. Analysis of PNA oligomers targeting SARS-CoV-2 RNA

### 3-1. Mass structure analysis and target binding affinity confirmation

Next, mass structure analysis was performed to confirm whether the designed PNA oligomers targeting SARS-CoV-2 were constructed as intended, and Tm analysis was performed to confirm the binding affinity of each PNA oligomer to the target. Each PNA oligomer designed in Table 2 above exhibited mass values that coincided with the predicted values at the time of design and the actually measured values, indicating that all were constructed as intended, and the following Table 8 provides some of the data. Meanwhile, Table 9 shows some of the results of the Tm measurement experiment to confirm the binding affinity of the PNA oligomer to the target. Since the 20-mer PNA oligomer showed high binding affinity even at 90°C or higher, the Tm value was measured using the 10-mer at the N-terminus and the 10-mer at the C-terminus of the PNA to compare the relative binding affinity of each PNA oligomer.

The Tm value for the duplex of 10-mer DNA complementary to PNA is so high that it is difficult to reliably measure the value in an aqueous buffer solution because the aqueous buffer solution tends to boil during the Tm measurement. Therefore, the Tm value was measured as follows using a UV/Vis spectrophotometer. In a 15 mL polypropylene Falcon tube, 4 µM PNA oligomer and 4 µM 10-mer DNA for the N-terminus were mixed in a buffer solution (pH 7.16, 10 mM sodium phosphate, 100 mM NaCl), then incubated at 90°C for 1 minute, and slowly cooled to room temperature. The solution was transferred to a 3 mL quartz cuvette, sealed well, and mounted on an Agilent Cary 100 UV/Visible spectrophotometer. The change in absorbance at 260 nm was measured while increasing the temperature of the cuvette at a rate of 0.5 or 1.0°C per minute, and the temperature at which the rate of change in absorbance was the greatest, that is, the inflection point, was determined as the Tm value between the PNA oligomer and DNA. The DNA used for Tm measurement was purchased from BIONEER CORPORATION (www.bioneer.com, Daejeon, Republic of Korea) and used without separate purification.

**[Table 8]**

| PNA oligomer | Exact Mass, m/z | |
|---|---|---|
| | the or.^{a} | obs.^{b} |
| PNA 35 | 6317.75 | 6317.9 |
| PNA 53 | 5419.37 | 5419.41 |
| PNA 76 | 6117.66 | 6117.7 |
| PNA 79 | 6144.64 | 6144.6 |
| PNA 81 | 6126.61 | 6126.6 |
| PNA 84 | 6126.67 | 6126.66 |
| PNA 88 | 5001.19 | 5001.25 |
| PNA 91 | 6108.65 | 6108.65 |
| PNA 105 | 4106.82 | 4106.92 |
| PNA 111 | 6067.65 | 6067.59 |
| PNA 120 | 6015.66 | 6015.6 |
| PNA 121 | 6112.72 | 6112.7 |
| PNA 123 | 6232.75 | 6232.73 |
| PNA 129 | 6059.63 | 6059.75 |
| PNA 143 | 6037.56 | 6037.52 |
| PNA 157 | 5419.37 | 5419.38 |
| a) Theoretical value, b) Observed value. | | |

**[Table 9]**

| PNA oligomer | Tm value (°C) | |
|---|---|---|
| | 10-mer DNA for N-terminus | 10-mer DNA for C-terminus |
| PNA 53 | 68.22 | 77.02 |
| PNA 76 | 79.27 | 63.02 |
| PNA 79 | 65.42 | 80.02 |
| PNA 81 | 59.02 | 60.02 |
| PNA 84 | 71.02 | 67.02 |
| PNA 88 | 59.02 | 69.07 |
| PNA 91 | 51.02 | 51.02 |
| PNA 120 | 70.42 | 48.02 |
| PNA 121 | 73.02 | 65.02 |
| PNA 123 | 77.52 | 71.07 |
| PNA 129 | 68.00 | 59.02 |
| PNA 143 | 66.02 | 52.02 |

### 3-2. Antiviral efficacy evaluation

The efficacy of drugs based on the constructed PNA oligomer was evaluated to determine how well the drugs could block viral protein production and gene replication.

Additionally, to confirm whether the antiviral efficacy is enhanced during treatment with a combination of multiple PNA oligomers, an experiment in which drugs (Multi) combined as shown in the following Table 10 were treated was also performed.

**[Table 10]**

| Multi drug name | Drug 1 | Drug 2 | Drug 3 | Drug 4 |
|---|---|---|---|---|
| Multi 1 | PNA 35 | PNA 76 | | |
| Multi 2 | PNA 129 | PNA 76 | | |
| Multi 3 | PNA 129 | PNA 79 | | |
| Multi 4 | PNA 129 | PNA 84 | | |
| Multi 5 | PNA 143 | PNA 76 | | |
| Multi 6 | PNA 143 | PNA 88 | | |
| Multi 7 | PNA 53 | PNA 120 | | |
| Multi 8 | PNA 35 | PNA 120 | | |
| Multi 9 | PNA 129 | PNA 120 | | |
| Multi 10 | PNA 143 | PNA 120 | | |
| Multi 11 | PNA 88 | PNA 120 | | |
| Multi 12 | PNA 76 | PNA 120 | | |
| Multi 13 | PNA 81 | PNA 120 | | |
| Multi 14 | PNA 79 | PNA 120 | | |
| Multi 15 | PNA 84 | PNA 120 | | |
| Multi 16 | PNA 91 | PNA 120 | | |
| Multi 17 | PNA 88 | PNA 121 | | |
| Multi 18 | PNA 76 | PNA 121 | | |
| Multi 19 | PNA 81 | PNA 121 | | |
| Multi 20 | PNA 79 | PNA 121 | | |
| Multi 21 | PNA 84 | PNA 121 | | |
| Multi 22 | PNA 91 | PNA 121 | | |
| Multi 23 | PNA 79 | PNA 123 | | |
| Multi 24 | PNA 105 | PNA 120 | | |
| Multi 25 | PNA 111 | PNA 120 | | |
| Multi 26 | PNA 120 | PNA 53 | PNA 79 | |
| Multi 27 | PNA 129 | PNA 53 | PNA 79 | |
| Multi 28 | PNA 120 | PNA 129 | PNA 53 | PNA 79 |
| Multi 29 | PNA 35 | PNA 76 | PNA 120 | |
| Multi 30 | PNA 129 | PNA 76 | PNA 120 | |
| Multi 31 | PNA 129 | PNA 79 | PNA 120 | |
| Multi 32 | PNA 129 | PNA 84 | PNA 120 | |
| Multi 33 | PNA 143 | PNA 76 | PNA 120 | |
| Multi 34 | PNA 143 | PNA 88 | PNA 120 | |
| Multi 35 | PNA 53 | PNA 76 | PNA 120 | |
| Multi 36 | PNA 53 | PNA 79 | PNA 123 | |

### 3-2-1. qRT-PCR

In order to analyze the level of viral RNA in viral particles present in a virus culture solution to determine how well the constructed PNA oligomer-based drugs inhibit viral genetic replication, infectious virus genes were quantified by performing qRT-PCR in a virus culture solution treated with a test substance (drug) at a concentration of 1000 nM.

For this purpose, Vero cells cultured in 12-well plates the day before were pre-incubated in a cell culture solution containing an evaluation drug at a set concentration (1000 nM) for 2 hours, and then infected with the virus, and the cell culture solution was treated with the evaluation drug at the same concentration. 24 hours after infection, viral RNA was extracted from the virus culture solution and quantified by qPR-PCR to compare and analyze the antiviral efficacy and consequently select a therapeutic substance. Paxlovid, used as a positive control, was also treated by the same method.

The screening results are shown in the following Table 11.

It could be confirmed that among the 50 PNA and Multi drugs, 20 drugs shown in bold in Table 11 had a virus inhibition rate equal to or higher than Paxlovid's 96.03%.

**[Table 11]**

| PNA oligomer | Inhibition% | PNA oligomer | Inhibition% |
|---|---|---|---|
| PNA 35 | 95.11 | Multi 12 | 95 |
| PNA 53 | 91.53 | Multi 13 | 77.95 |
| PNA 76 | 81.01 | Multi 14 | 79.61 |
| PNA 79 | 88.62 | Multi 15 | 95.98 |
| PNA 81 | 58.46 | Multi 16 | 85.98 |
| PNA 84 | 87.52 | Multi 17 | 93.29 |
| PNA 88 | 79.03 | **Multi 18** | **96.78** |
| PNA 91 | 65.14 | Multi 19 | 94.21 |
| PNA 120 | 50.95 | Multi 20 | 94.82 |
| PNA 121 | 71.48 | **Multi 21** | **96.09** |
| **PNA 123** | **97.44** | Multi 22 | 95.53 |
| PNA 129 | 95.34 | **Multi 23** | **98.49** |
| PNA 143 | 48.5 | Multi 24 | 84.16 |
| PNA 157 | 90.07 | Multi 25 | 82.07 |
| Multi 1 | 96.94 | **Multi 26** | **97.55** |
| **Multi 2** | **97.84** | **Multi 27** | **97.95** |
| **Multi 3** | **96.57** | **Multi 28** | **98.15** |
| **Multi 4** | **96.51** | **Multi 29** | **98.5** |
| Multi 5 | 94.16 | **Multi 30** | **97.46** |
| Multi 6 | 95.83 | **Multi 31** | **96.03** |
| **Multi 7** | **96.31** | **Multi 32** | **96.16** |
| **Multi 8** | **97.81** | Multi 33 | 95.25 |
| Multi 9 | 95.67 | **Multi 34** | **97.7** |
| Multi 10 | 89.35 | **Multi 35** | **96.5** |
| Multi 11 | 80.04 | **Multi 36** | **97.4** |
| | | Paxlovid | 96.03 |

### 3-2-2. Titer analysis

The 50% inhibitory concentration (IC50) was calculated for the 20 drugs in Example 3-2-1, which exhibited excellent virus proliferation inhibition efficacy.

To this end, Vero cells were cultured in a 12-well plate, and pre-incubation was performed in a cell culture solution containing an evaluation drug at each concentration for 2 hours. Thereafter, cells were infected with the virus, and the evaluation drug at the same concentration was added to the cell culture solution for each well. After 24 hours, viral RNA was extracted from the virus culture solution and quantified by qPR-PCR to analyze the antiviral efficacy. The treatment concentration of the test substance used in the experiment was set as shown in the following Table 12, and a 2-fold dilution was used.

**[Table 12]**

| Well | 1 | 2 | 3 | 4 | 5 | 6 |
|---|---|---|---|---|---|---|
| Concentration (nM) | 1000 | 500 | 250 | 125 | 62.5 | 31.25 |

| Well | 7 | 8 | 9 | 10 | 11 | 12 |
|---|---|---|---|---|---|---|
| Concentration (nM) | 15.63 | 7.81 | 3.91 | 1.95 | 0.98 | 0.49 |

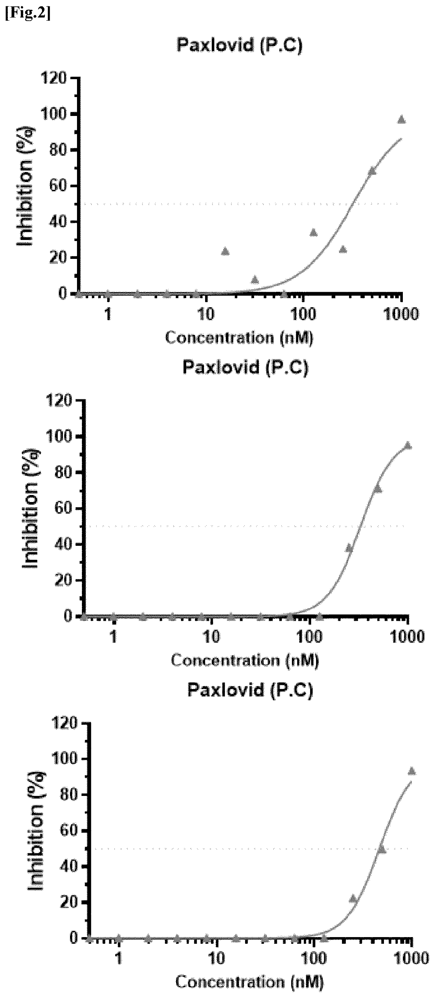

The results of three repeated experiments are shown in the following Table 13 and FIGS. 2 to 3B.

**[Table 13]**

| PNA derivative | IC50 (nM) |
|---|---|
| PNA 123 | 263.70 |
| Multi 1 | 327.70 |
| Multi 2 | 375.20 |
| Multi 3 | 434.40 |
| Multi 4 | 501.90 |
| Multi 7 | 323.60 |
| Multi 8 | 342.60 |
| Multi 18 | 690.10 |
| Multi 21 | 481.10 |
| Multi 23 | 287.10 |
| Multi 26 | 218.30 |
| Multi 27 | 215.10 |
| Multi 28 | 61.54 |
| Multi 29 | Ambiguous |
| Multi 30 | 262.60 |
| Multi 31 | 316.30 |
| Multi 32 | 370.40 |
| Multi 34 | 275.70 |
| Multi 35 | 432.50 |
| Multi 36 | 216.60 |
| Paxlovid (P.C)-1 | 316.60 |
| Paxlovid (P.C)-2 | 328.30 |
| Paxlovid (P.C)-3 | 464.40 |

The IC50 of Paxlovid used as a positive control in each experiment was confirmed to be 316.6, 328.3, and 464.4 nM, respectively (FIG. 2).

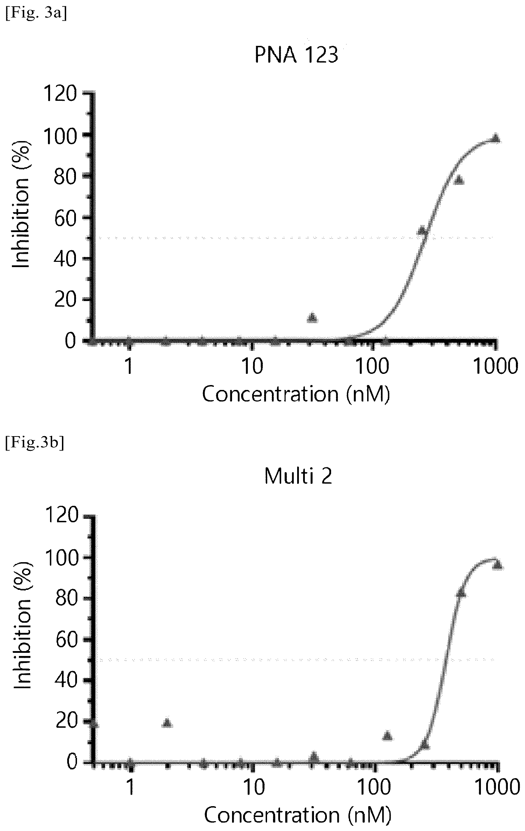

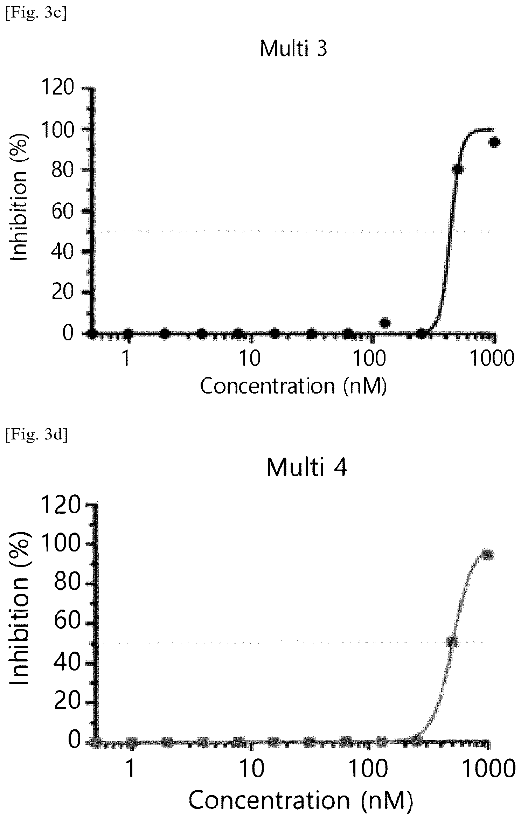

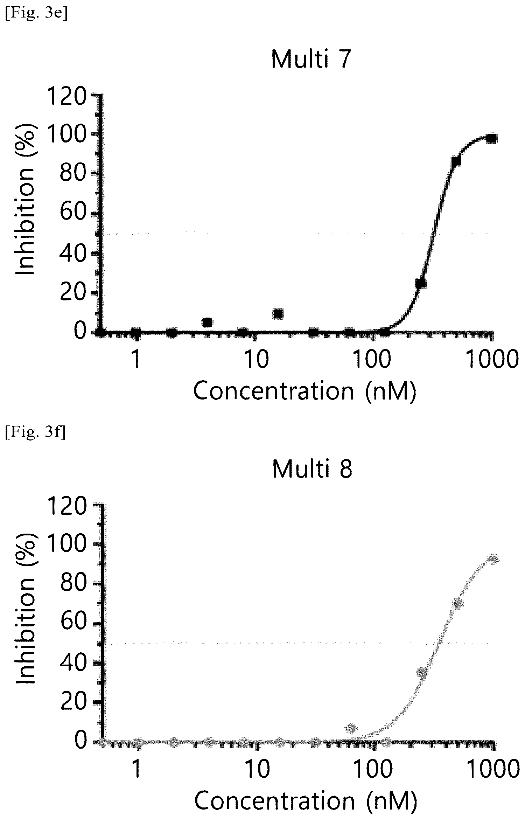

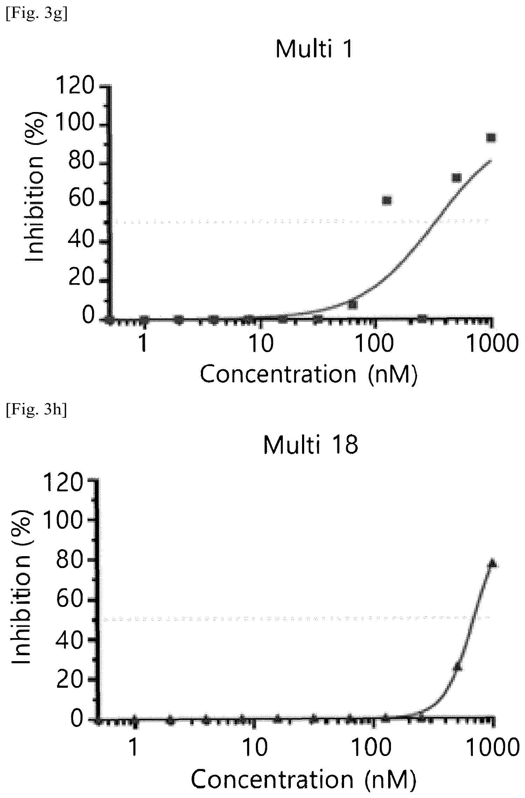

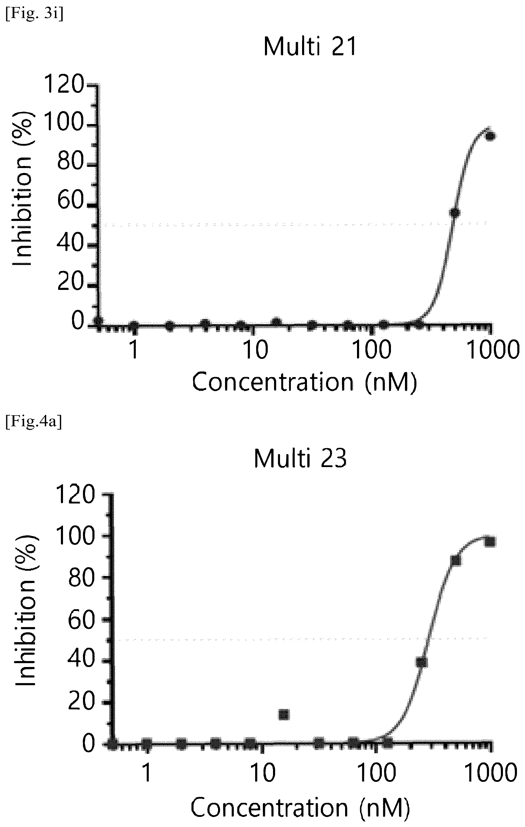

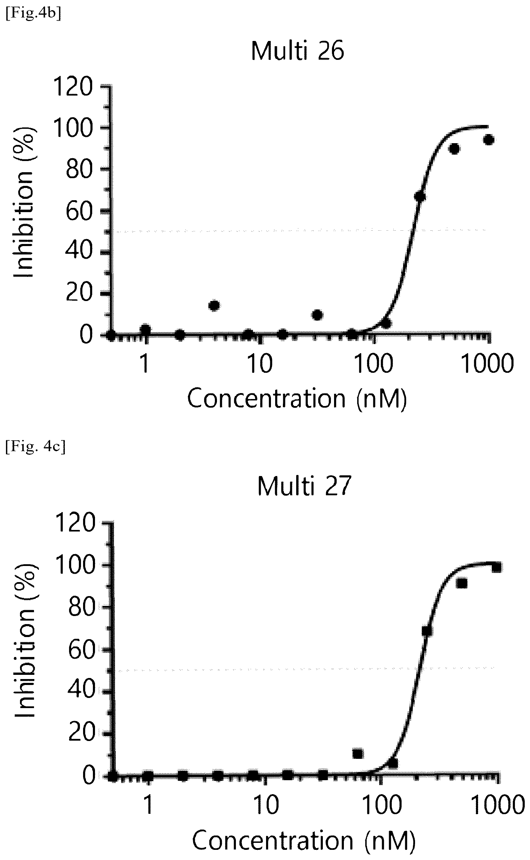

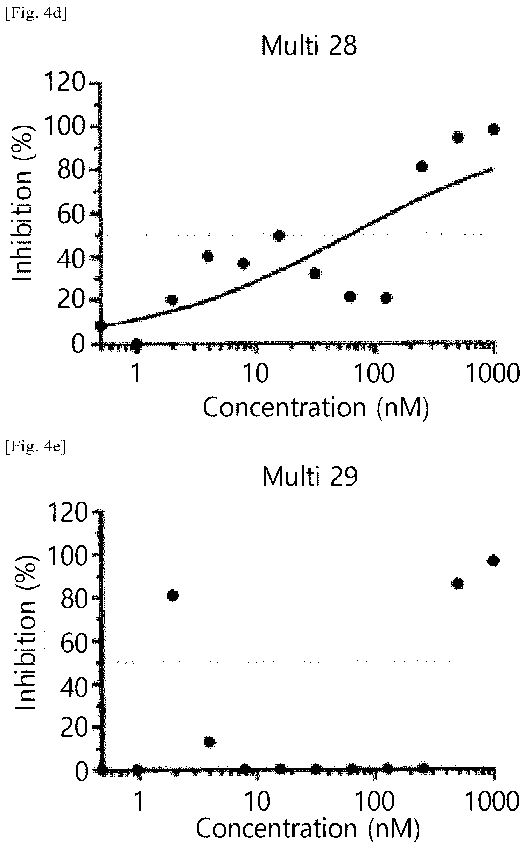

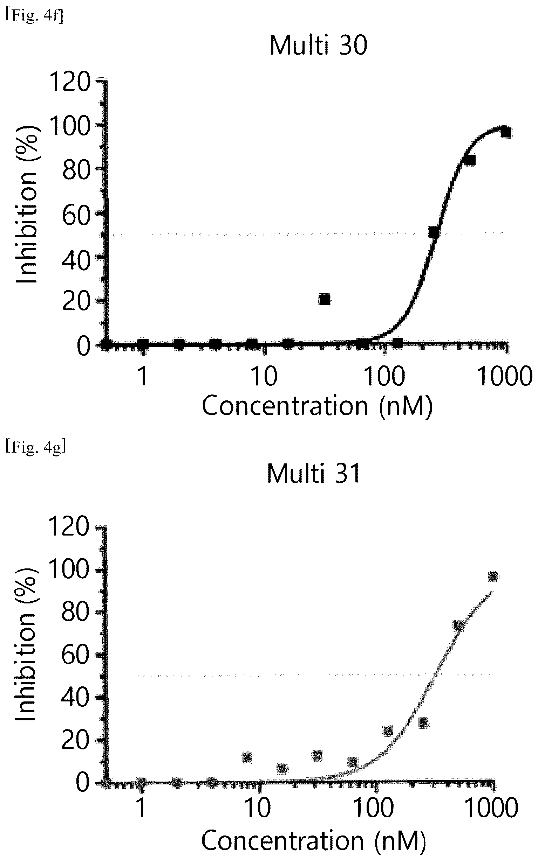

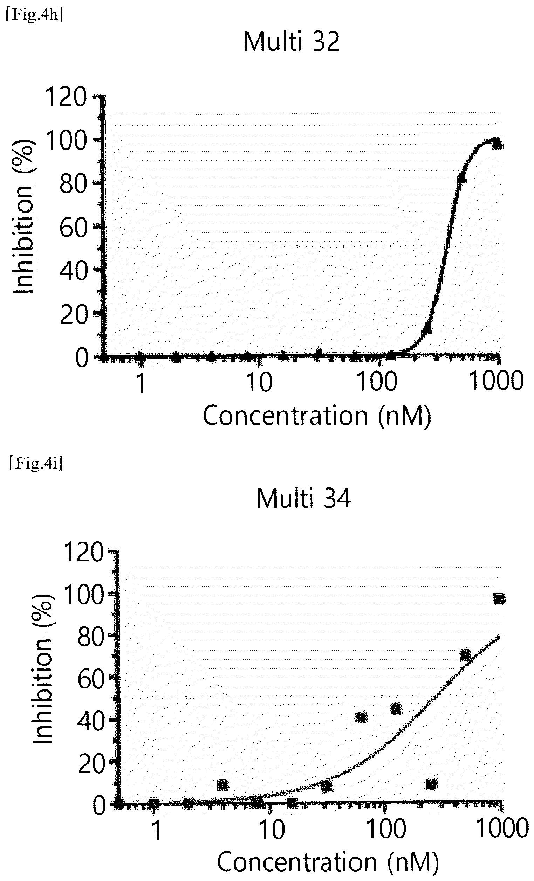

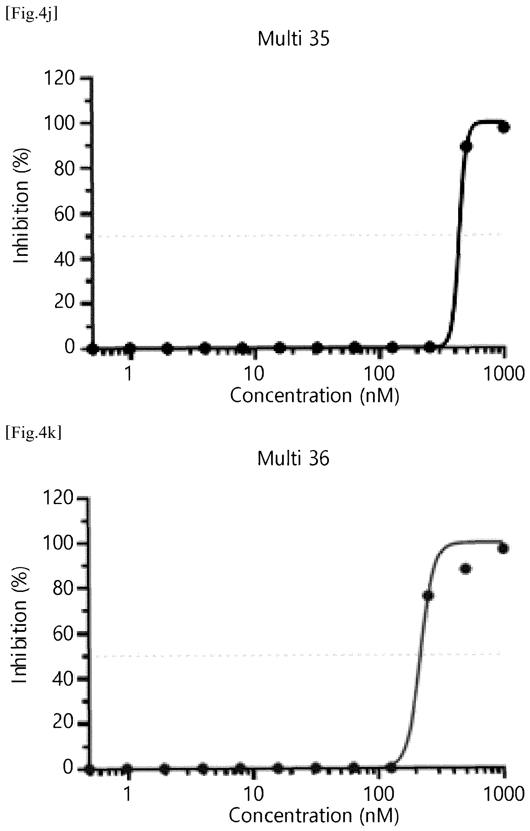

As a result of deriving the IC50, it was confirmed once again that 19 out of 20 types had a virus inhibition rate of 90% or more at 1000 nM. In addition, it was confirmed that when the concentration of the test substance was below 250 nM, the inhibition rate did not exceed 50% or the antiviral efficacy disappeared (FIGS. 3A to 3I and FIGS. 4A to 4K).

Multi 18 drug was the only one that was confirmed to have a virus inhibition rate of 90% or less at 1000 nM. Moreover, since Multi 28 and Multi 29 did not show a dose-dependent pattern, the IC50 was very low or not derived, i.e., derived as 61.54 nM and Ambiguous, respectively. Therefore, the IC50 was not derived from these three substances for re-verification.

As a result of deriving the IC50 for 17 substances except for the 3 excluded substances, the IC50 range of the test substances was confirmed to be 215.1 to 690.1 nM (Table 9). Among them, the IC50 of Multi 27, Multi 36, and Multi 26 was confirmed to be the best. As a single drug, PNA 123 was also confirmed to show a better IC50 than Paxlovid.

### Example 4. SARS-CoV-2 proliferation inhibition effect of PNA oligomers targeting HCoV-OC43 RNA

To confirm whether viral replication can be inhibited even when there is a mismatch in the complementary binding region between the PNA oligomer and the target gene, it was confirmed whether PNA oligomers targeting HCoV-OC43 RNA inhibited the protein translation of the SARS-CoV-2 virus. Accordingly, a SARS-CoV-2 virus culture solution was treated with three types of PNA oligomers (PNA69, 53, 46) designed to be complementary to a portion of HCoV-OC43 RNA, and western blotting was performed to confirm the level of expression of the N protein of the virus.

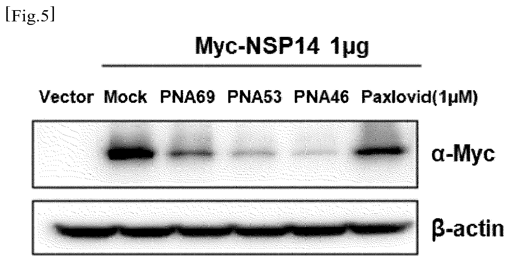

Specifically, for drug screening, HCT8 cells cultured in 6-well plates the day before were treated with 1 µM PNA derivatives while replacing the medium with fresh medium. Thereafter, Myc-NSP14 was transfected according to the manual using Lipofectamine 2000. 48 hours after transfection, the cells were harvested, lysed with an M-PER lysis solution, and centrifuged, and then a western blot sample was produced from the supernatant using a 4x sample buffer. Analysis was performed on a 12% SDS-PAGE gel. The antibodies used were anti-myc (Cell Signaling Technology) and anti-β-actin (Merck). Detection was performed using ECL Detection Reagent (AbClon), visualization and analysis were performed using Amersham ImageQuant 800, and the results are shown in FIG. 5.

Although the embodiments have been described with limited drawings as described above, those skilled in the art can apply various technical modifications and variations from the above description. For example, even though the described techniques are performed in a different order from the order described above, and/or the described components such as systems, structures, devices, and circuits are combined in a different manner from the manner described above, or the components are replaced or substituted with other components or equivalents thereof, appropriate results can be achieved.

Therefore, other implementations, other embodiments, and equivalents to the claims also belong to the scope of the claims to be described below.

### [Industrial Applicability]

The modified PNA oligomer of the present invention can be used as an effective coronavirus therapeutic agent that can respond to rapidly mutating viral infectious diseases.

## Claims

1. A pharmaceutical composition for preventing or treating coronavirus infectious diseases, comprising a peptide nucleic acid (PNA) oligomer represented by the following Chemical Formula 1 or a pharmaceutically acceptable salt thereof as an active ingredient,
wherein the PNA oligomer is able to complementarily bind to a portion of the genomic (+) RNA or genomic (-) RNA of coronavirus: 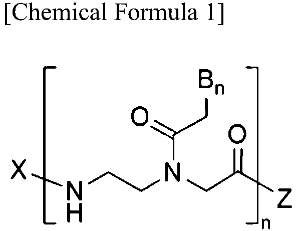
in the formula,
n is an integer between 10 and 30;
X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and a substituted or non-substituted arylsulfonyl;
Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl;
B₁, B₂, ......, Bₙ₋₁ and Bₙ are each independently selected from a natural nucleotide and a non-natural nucleotide of adenine (A), guanine (G), thymine (T), or cytosine (C);
one or more of B₁ to Bₙ are each independently selected from the group consisting of modified non-natural nucleobases of the following Chemical Formulas 2 to 4; 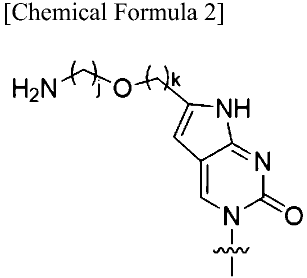 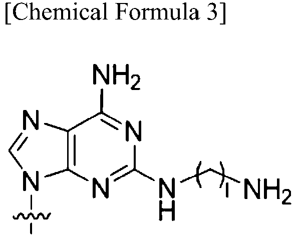 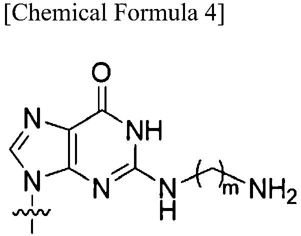
in Chemical Formulae 2 to 4, j, k, l, and m are each independently an integer between 1 and 16.

2. The pharmaceutical composition of claim 1, wherein the pharmaceutical composition does not comprise a delivery vehicle for intracellular permeation of the PNA oligomer.

3. The pharmaceutical composition of claim 1, wherein X is Fmoc [(9-fluorenyl)methyloxy]carbonyl], Fethoc [2-(9-fluorenyl)ethyl-1-oxy]carbonyl], acetyl (Ac), benzoyl, or hydrogen.

4. The pharmaceutical composition of claim 1, wherein X is Fethoc [2-(9-fluorenyl)ethyl-1-oxy]carbonyl],
Z is NH2,
j is 2,
k is 1,
l is 5, and
m is 6.

5. The pharmaceutical composition of claim 1, wherein the coronavirus is one or more selected from the group consisting of human coronavirus 229E (HCoV-229E), human coronavirus OC43 (HCoV-OC43), severe acute respiratory syndrome coronavirus (SARS-CoV), human coronavirus NL63 (HCoV-NL63), human coronavirus HKU1 (HCoV-HKU1), Middle East respiratory syndrome coronavirus (MERS-CoV), and severe acute respiratory syndrome coronavirus type 2 (SARS-Cov-2).

6. The pharmaceutical composition of claim 1, wherein the coronavirus is human coronavirus OC43 (HCoV-OC43), and
the PNA oligomer is one or more selected from the group consisting of PNA oligomers in Table 1.

7. The pharmaceutical composition of claim 1, wherein the coronavirus is severe acute respiratory syndrome coronavirus type 2 (SARS-Cov-2), and
the PNA oligomer is one or more selected from the group consisting of PNA oligomers in Table 2.

8. The pharmaceutical composition of claim 7, wherein the pharmaceutical composition comprises PNA 123 [Fethoc-A(5)GC-C(1O2)CT-G(6)TA-TA(5)C-GA(5)C-ATC(1O2)-AG-NH2] in Table 2.

9. The pharmaceutical composition of claim 1, wherein the coronavirus is one or more selected from the group consisting of human coronavirus OC43 (HCoV-OC43) and severe acute respiratory syndrome coronavirus type 2 (SARS-Cov-2), and
the PNA oligomer is one or more selected from the group consisting of PNA oligomers in Table 3.

10. The pharmaceutical composition of claim 1, wherein the PNA oligomer is 80% or more complementary to a portion of the genomic (+) RNA or genomic (-) RNA of coronavirus.

11. The pharmaceutical composition of claim 1, wherein the PNA oligomer is 90% or more complementary to a portion of the genomic (+) RNA or genomic (-) RNA of coronavirus.

12. A method for preventing or treating coronavirus infectious diseases, the method comprising administering a peptide nucleic acid (PNA) oligomer represented by the following Chemical Formula 1 or a pharmaceutically acceptable salt thereof to an individual,
wherein the PNA oligomer is able to complementarily bind to a portion of the genomic (+) RNA or genomic (-) RNA of coronavirus: 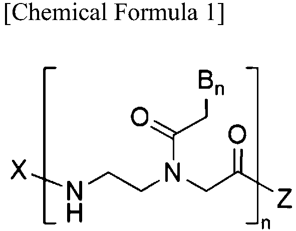
in the formula,
n is an integer between 10 and 30;
X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and a substituted or non-substituted arylsulfonyl;
Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl;
B₁, B₂, ......, Bₙ₋₁ and Bₙ are each independently selected from a natural nucleotide and a non-natural nucleotide of adenine (A), guanine (G), thymine (T), or cytosine (C);
one or more of B₁ to Bₙ are each independently selected from the group consisting of modified non-natural nucleobases of the following Chemical Formulas 2 to 4; 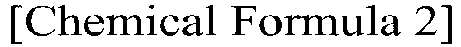 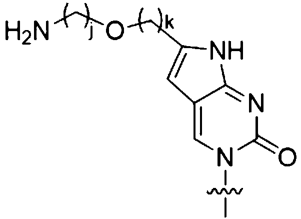 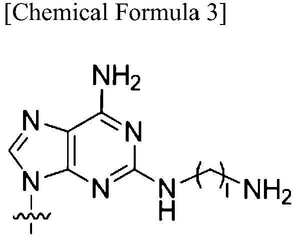 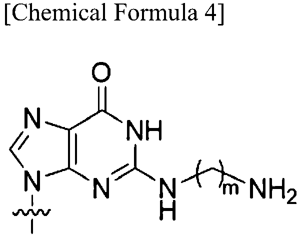
in Chemical Formulae 2 to 4, j, k, 1, and m are each independently an integer between 1 and 16.

13. A method for inhibiting coronavirus proliferation, the method comprising administering a peptide nucleic acid (PNA) oligomer represented by the following Chemical Formula 1 or a pharmaceutically acceptable salt thereof to an individual,
wherein the PNA oligomer is able to complementarily bind to a portion of the genomic (+) RNA or genomic (-) RNA of coronavirus: 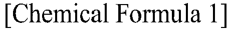 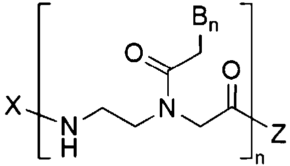
in the formula,
n is an integer between 10 and 30;
X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and a substituted or non-substituted arylsulfonyl;
Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl;
B₁, B₂, ......, Bₙ₋₁ and Bₙ are each independently selected from a natural nucleotide and a non-natural nucleotide of adenine (A), guanine (G), thymine (T), or cytosine (C);
one or more of B₁ to Bₙ are each independently selected from the group consisting of modified non-natural nucleobases of the following Chemical Formulas 2 to 4; 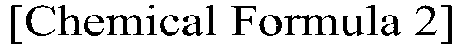 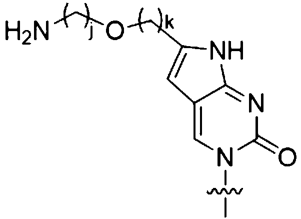 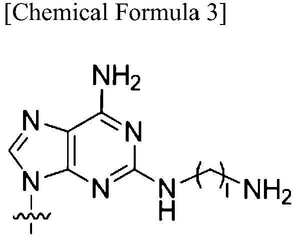 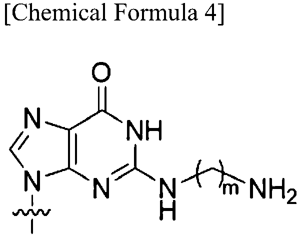
in Chemical Formulae 2 to 4, j, k, 1, and m are each independently an integer between 1 and 16.

14. A use of a peptide nucleic acid (PNA) oligomer represented by the following Chemical Formula 1 or a pharmaceutically acceptable salt thereof for preparing a drug for preventing or treating coronavirus infectious diseases,
wherein the PNA oligomer is able to complementarily bind to a portion of the genomic (+) RNA or genomic (-) RNA of coronavirus: 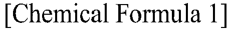 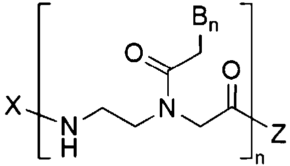
in the formula,
n is an integer between 10 and 30;
X is one selected from the group consisting of hydrogen [H], formyl [H-C(=O)-], aminocarbonyl [NH2-C(=O)-], a substituted or non-substituted alkyl, a substituted or non-substituted aryl, a substituted or non-substituted alkylacyl, a substituted or non-substituted arylacyl, a substituted or non-substituted alkyloxycarbonyl, a substituted or non-substituted aryloxycarbonyl, a substituted or non-substituted alkylaminocarbonyl, a substituted or non-substituted arylaminocarbonyl, a substituted or non-substituted alkylsulfonyl, and a substituted or non-substituted arylsulfonyl;
Z is one selected from the group consisting of hydroxy [-OH], a substituted or non-substituted alkyloxy, a substituted or non-substituted aryloxy, amino [-NH2], a substituted or non-substituted alkylamino, a substituted or non-substituted arylamino, a substituted or non-substituted alkyl, and a substituted or non-substituted aryl;
B₁, B₂, ......, Bₙ₋₁ and Bₙ are each independently selected from a natural nucleotide and a non-natural nucleotide of adenine (A), guanine (G), thymine (T), or cytosine (C);
one or more of B₁ to Bₙ are each independently selected from the group consisting of modified non-natural nucleobases of the following Chemical Formulas 2 to 4; 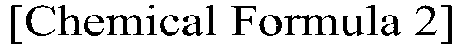 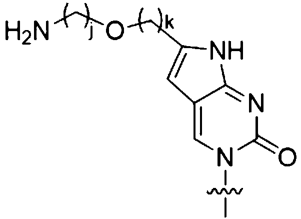 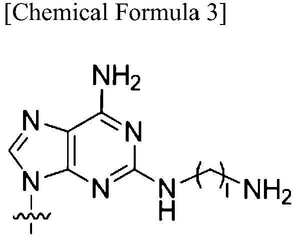 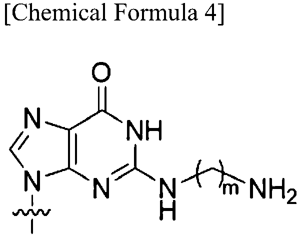
in Chemical Formulae 2 to 4, j, k, 1, and m are each independently an integer between 1 and 16.
